# EUROPEAN PATENT APPLICATION

(11) **EP 4 501 915 A1**
(43) Date of publication of application: **05.02.2025**
(21) Application number: 23781385.2
(22) Date of filing: 30.03.2023
(51) Int. Cl.: C07D 235/32, C07D 491/113, A61K 31/496, A61K 31/438, A61K 31/541, C07D 405/12, C07D 295/096, C07D 307/64, A61K 31/454, A61K 31/5377

(54) **THIOBENZIMIDAZOLE DERIVATIVE PREPARATION METHOD AND NOVEL INTERMEDIATE THEREFOR**

(30) Priority: 30.03.2022 KR 20220039908; 30.03.2022 KR 20220039910
(71) Applicant: Korea University Research and Business Foundation, Seoul 02841 (KR)
(72) Inventor: SEO, Jae Hong, Gwacheon-si Gyeonggi-do 13803 (KR); NAM, Kee Dal, Seoul 02467 (KR); KIM, Ji Young, Seoul 08307 (KR); KIM, Yoon Jae, Paju-si Gyeonggi-do 10907 (KR); KANG, Yong Koo, Seoul 08281 (KR)
(74) Representative: Weickmann & Weickmann PartmbB
(86) International application number: PCT/KR2023/004255
(87) International publication number: WO 2023/191533

(57) **Abstract**

The present invention relates to a thiobenzimidazole derivative preparation method, a novel intermediate therefor, and the like. The present invention uses an intermediate containing O-ethyl carbonodithioate or an intermediate containing methyl 3-mercaptopropionate so as to effectively synthesize a thiobenzimidazole derivative having anti-cancer activity.

## Description

### TECHNICAL FIELD

The present disclosure relates to a method for preparing a thiobenzimidazole derivative, a novel intermediate therefor, and the like.

### BACKGROUND ART

Patients with triple-negative breast cancer (TNBC; ER-, PR-, HER2-), who account for 10-15% of all breast cancer patients, lack hormone receptors (ER (estrogen receptors), PR (progesterone receptors) and HER2 proteins, and thus they do not benefit from hormone therapy or HER2-targeted therapy. Currently, the standard of care for triple-negative breast cancer relies entirely on general cytotoxic anti-cancer drugs (Taxene- or anthracene-based agents). Since there are no established targeted therapeutic agents, treatment strategies for other subtypes are less diverse than those for breast cancer. More seriously, after surgery or anticancer treatment, recurrence occurs within 2-3 years in most patients, and metastasis to other organs such as lung, liver, brain and bone is easily triggered, affecting the survival rate of the patients.

The 5-year overall survival rate for patients diagnosed with stage III cancer is less than 55%, and for patients whose cancer has already metastasized (advanced-stage), the 5-year overall survival rate is even lower, at 30% or less. This is a very serious disease that will ultimately kill most of these patients within a few years.

Microtubules are a major component of the cytoskeleton and are made up of tubulin heteropolymers, which are composed of α and β subunits. Microtubules perform a variety of cellular functions, including intracellular transport, polarity maintenance, intracellular signaling, and cell migration and proliferation. During cell mitosis, microtubles form spindle fibers, which arrange chromosomes at the center of the cell and then separate them into two poles. If the spindle fibers fail to function properly, cell division is inhibited and apoptosis occurs, making the microtuble an attractive target for anti-cancer drugs.

Drugs targeting microtubules are largely divided into two groups: those that stabilize microtubules and those that destabilize microtubules. First, microtubule stabilizers include taxane, paclitaxel (Taxol), decetaxel, and the like, which serve to prevent microtubules from depolymerization and to enhance polymerization. Most of the microtubule stabilizers bind to either the taxane-binding site or the overlapping site of β-tubulin. Second, microtubule destabilizers include colchicines, vinca alkaloids, and the like, which bind to the colchicine binding site or the vinca binding site. Drugs targeting microtubules themselves are more effective at lower concentrations than drugs affecting microtubule polymers, and the result is the same: they inhibit cell mitosis. Therefore, there is a need to develop a tubulin polymerization inhibitor with potential as an anticancer agent.

On the other hand, flubendazole and albendazole, which are also tubulin polymerization inhibitors, have been commercially available as anthelmintic drugs, but they have been attracting attention as a treatment for various cancers because they may cause cell death by cell cycle arrest, and may also have a killing effect onslow-growing cancer cells. However, there is a limitation that in vivo absorption is difficult due to their low solubility in water, and side effects such as elevated liver enzyme levels appear when they are consumed in large amounts, and thus the development of derivatives with new structures is required.

Accordingly, the present inventors confirmed that a novel thiobenzimidazole derivative or a pharmaceutically acceptable salt thereof induces apoptosis by stopping the cell cycle of cancer cells (cell cycle arrest) as a tubulin polymerization inhibitor, and developed a derivative and a method for producing the same used to produce the present derivative to complete the present disclosure.

### DISCLOSURE OF THE INVENTION

### TECHNICAL GOALS

The technical goal to be achieved by the present disclosure is to provide a method for producing a thiobenzimidazole derivative and an intermediate therefor.

Additionally, another aspect of the present disclosure is to provide a method for producing a pharmaceutically acceptable salt of a thiobenzimidazole derivative.

However, the technical goals to be achieved by the present disclosure are not limited to those mentioned above, and other goals not mentioned will be clearly understood by one of ordinary skill in the art from the following description.

### TECHNICAL SOLUTIONS

In order to achieve the above mentioned goals, the present disclosure provides a method for preparing a thiobenzimidazole derivative represented by [Chemical Formula 1], including the following steps:
(1) preparing a compound represented by [Chemical Formula 3] by adding potassium O-ethylcarbonodithioate to a compound represented by [Chemical Formula 2];
(2) preparing a compound represented by [Chemical Formula 4] by adding 5-chloro-2-nitroaniline to the compound represented by [Chemical Formula 3] as prepared above; and
(3) preparing a compound represented by [Chemical Formula 1] by adding 1,3-bis(methoxycarbonyl)-2-methyl 2-thiopseudoeura to the compound represented by [Chemical Formula 4] as prepared above.

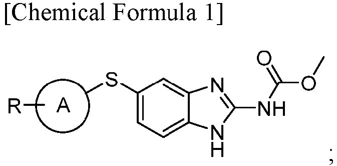

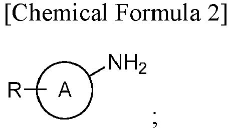

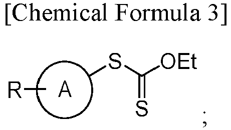

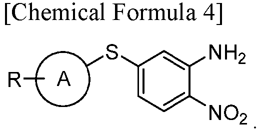

In an embodiment of the present disclosure, in Chemical Formulae 1 to 4, A may be a C₃-C₁₀ cyclic alkyl group, a heterocycloalkyl group, an aryl group, or a heteroaryl group, and R may be one or more selected from the group consisting of hydrogen, a halogen group, a chained or cyclic alkyl group of C₁-C₁₀, a heterocycloalkyl group, an aryl group, and a heteroaryl group, but is not limited thereto. The cyclic alkyl group, the heterocycloalkyl group, the aryl group, or the heteroaryl group may be unsubstituted or substituted with one or more selected from the group consisting of a C₁-C₁₀ chained alkyl group, an alkylsulfonyl group, and a carbonyl group, and the carbonyl group may be substituted with a chained or cyclic alkyl group of C₁-C₁₀, a heterocycloalkyl group, an aryl group, or a heteroaryl group, but is not limited thereto.

In another embodiment of the present disclosure, A may be one or more selected from the group consisting of a phenyl group, a furanyl group, a pyridinyl group, and a pyrimidinyl group, and R may be one or more selected from the group consisting of hydrogen, a halogen group, a methyl group, an ethyl group, a propyl group, a piperazinyl group, a piperidinyl group, a morpholinyl group, a thiomorpholinyl group, a thiophenyl group, an imidazolyl group, a triazolyl group, and 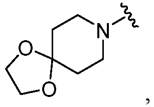 but is not limited thereto. The piperazinyl or piperidinyl group may be unsubstituted or substituted with one or more selected from the group consisting of a methyl group, an ethyl group, a propyl group, a methylsulfone group, and 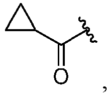 but is not limited thereto.

In another embodiment of the present disclosure, the compound represented by [Chemical Formula 1] may be any one selected from the group consisting of the following [Chemical Formula 1-1] to [Chemical Formula 1-7]: 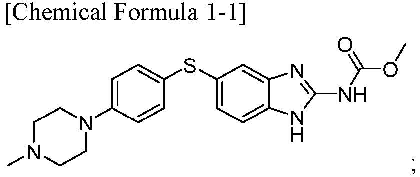 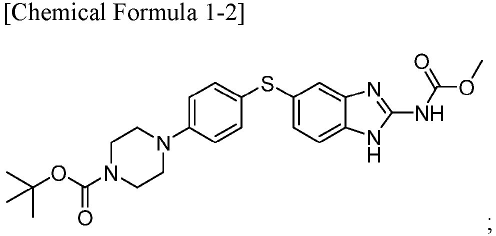 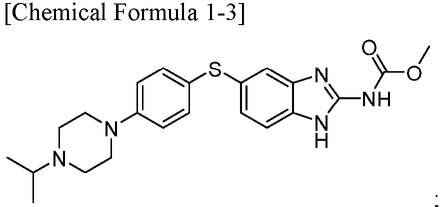 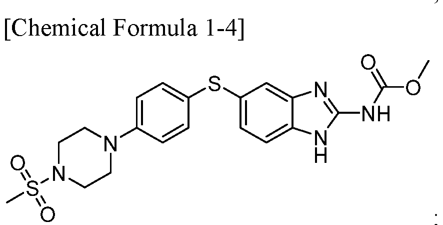 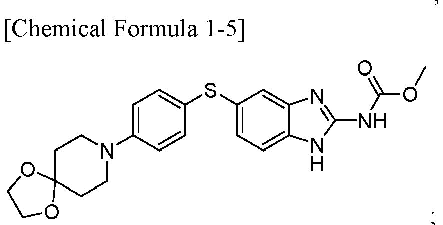 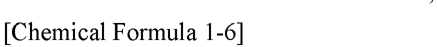 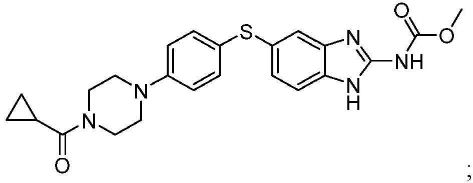 and

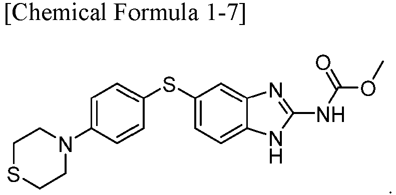

In another embodiment of the present disclosure, the compound represented by [Chemical Formula 2] may be any one selected from the group consisting of the following [Chemical Formula 2-1] to [Chemical Formula 2-7]: 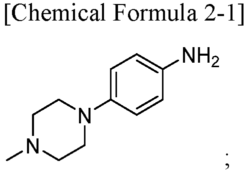 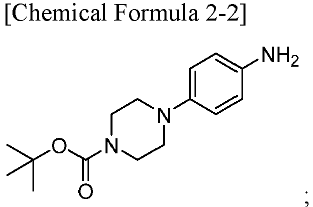 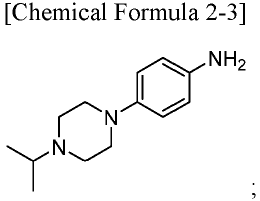 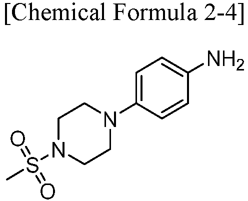 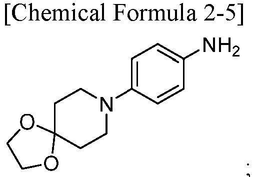 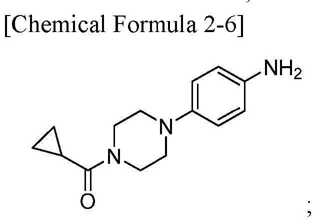 and

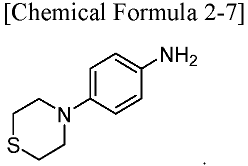

In another embodiment of the present disclosure, the compound represented by [Chemical Formula 3] may be any one selected from the group consisting of the following [Chemical Formula 3-1] to [Chemical Formula 3-7]: 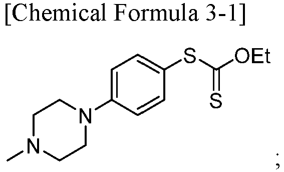 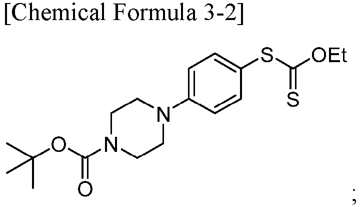 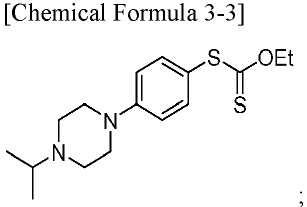 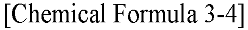 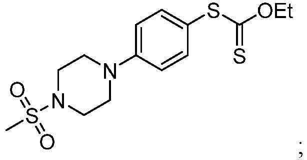 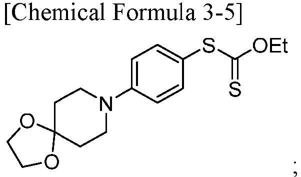 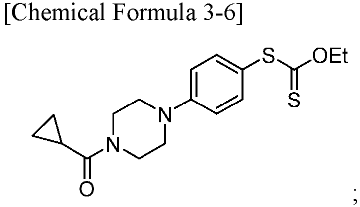 and

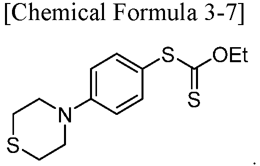

In another embodiment of the present disclosure, the compound represented by [Chemical Formula 4] may be any one selected from the group consisting of the following [Chemical Formula 4-1] to [Chemical Formula 4-7]: 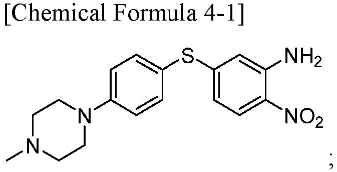 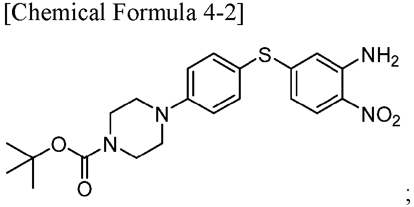 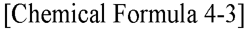 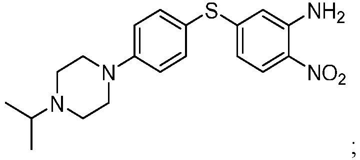 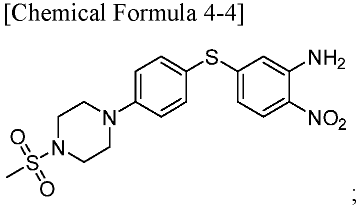 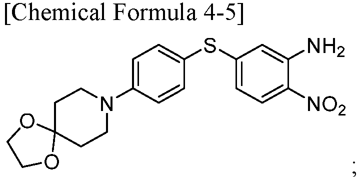 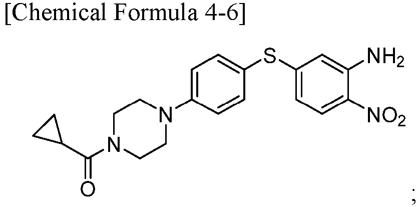 and

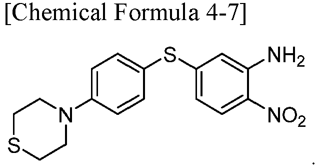

In another embodiment of the present disclosure, step (1) may be performed by adding sulfuric acid (H₂SO₄) and sodium nitrite (NaNO₂) at -5 to 0°C and performing stirring for 2 hours, and then adding potassium O-ethylcarbonodithioate at room temperature and performing stirring for 2 hours.

In another embodiment of the present disclosure, step (2) may be performed by adding sodium methoxide (MeONa) or methanol (MeOH) together with 5-chloro-2-nitroaniline to prepare a compound represented by [Chemical Formula 4] without reduction. Preferably, step (2) may be performed by adding sodium methoxide (MeONa) and performing stirring at 55°C for 16 hours, or by adding methanol (MeOH) and performing stirring at 65°C for 16 hours.

In another embodiment of the present disclosure, step (3) may be performed by adding acetic acid (AcOH) and zinc (Zn) together with 1,3-bis(methoxycarbonyl)-2-methyl 2-thiopseudoeura.

In another embodiment of the present disclosure, step (3) may be performed by synthesizing the following [Chemical Formula 5] from the compound represented by [Chemical Formula 4], and then adding 1,3-bis(methoxycarbonyl)-2-methyl 2-thiopseudoeura to prepare the compound represented by [Chemical Formula 1].

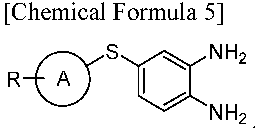

In an embodiment of the present disclosure, in Chemical Formula 5, A may be a C₃-C₁₀ cyclic alkyl group, a heterocycloalkyl group, an aryl group, or a heteroaryl group, and R may be one or more selected from the group consisting of hydrogen, a halogen group, a chained or cyclic alkyl group of C₁-C₁₀, a heterocycloalkyl group, an aryl group, and a heteroaryl group, but is not limited thereto. The cyclic alkyl group, the heterocycloalkyl group, the aryl group, or the heteroaryl group may be unsubstituted or substituted with one or more selected from the group consisting of a C₁-C₁₀ chained alkyl group, an alkylsulfonyl group, and a carbonyl group, and the carbonyl group may be substituted with a chained or cyclic alkyl group of C₁-C₁₀, a heterocycloalkyl group, an aryl group, or a heteroaryl group, but is not limited thereto.

In another embodiment of the present disclosure, the compound represented by [Chemical Formula 5] may be any one selected from the group consisting of the following [Chemical Formula 5-1] and [Chemical Formula 5-3] to [Chemical Formula 5-7]: 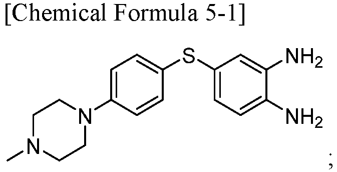 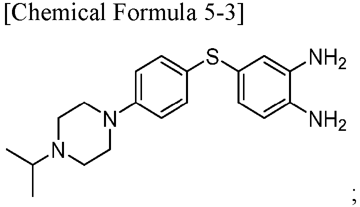 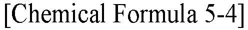 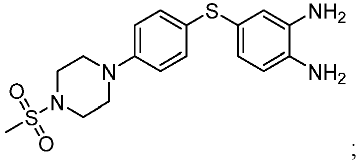 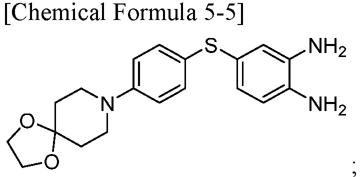 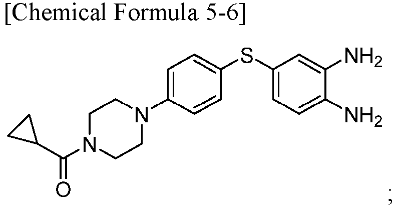 and

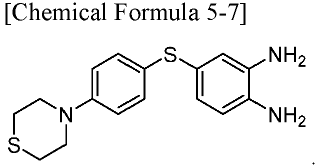

In another embodiment of the present disclosure, step (3) may be performed by adding acetic acid (AcOH) and zinc (Zn) to the compound represented by [Chemical Formula 4] to synthesize the compound represented by [Chemical Formula 5].

In another embodiment of the present disclosure, step (3) may be performed by adding methanol (MeOH) and palladium/carbon (Pd/C) to the compound represented by [Chemical Formula 4] to synthesize the compound represented by [Chemical Formula 5].

In another embodiment of the present disclosure, step (3) may be performed by adding ethanol (EtOH) and tin chloride (SnCl₂) to the compound represented by [Chemical Formula 4] to synthesize the compound represented by [Chemical Formula 5].

In addition, the present disclosure provides a method for preparing a pharmaceutically acceptable salt of the thiobenzimidazole derivative represented by [Chemical Formula 1], including the following steps:
(1) preparing a compound represented by [Chemical Formula 3] by adding potassium O-ethylcarbonodithioate to a compound represented by [Chemical Formula 2];
(2) preparing a compound represented by [Chemical Formula 4] by adding 5-chloro-2-nitroaniline to the compound represented by [Chemical Formula 3] as prepared above;
(3) preparing a compound represented by [Chemical Formula 1] by adding 1,3-bis(methoxycarbonyl)-2-methyl 2-thiopseudoeura to the compound represented by [Chemical Formula 4] as prepared above; and
(4) preparing a hydrogen chloride (HCl) salt of the compound represented by [Chemical Formula 1] by adding HCl to the compound represented by [Chemical Formula 1] as prepared above.

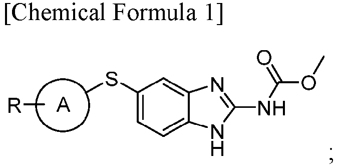

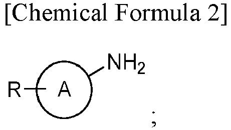

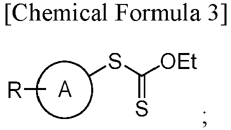

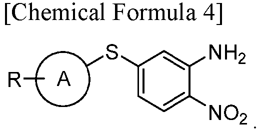

In an embodiment of the present disclosure, in Chemical Formulae 1 to 4, A may be a C₃-C₁₀ cyclic alkyl group, a heterocycloalkyl group, an aryl group, or a heteroaryl group, and R may be one or more selected from the group consisting of hydrogen, a halogen group, a chained or cyclic alkyl group of C₁-C₁₀, a heterocycloalkyl group, an aryl group, and a heteroaryl group, but is not limited thereto. The cyclic alkyl group, the heterocycloalkyl group, the aryl group, or the heteroaryl group may be unsubstituted or substituted with one or more selected from the group consisting of a C₁-C₁₀ chained alkyl group, an alkylsulfonyl group, and a carbonyl group, and the carbonyl group may be substituted with a chained or cyclic alkyl group of C₁-C₁₀, a heterocycloalkyl group, an aryl group, or a heteroaryl group, but is not limited thereto.

In another embodiment of the present disclosure, step (4) may be performed by adding methanol (MeOH) and hydrogen chloride (HCl) to the compound represented by [Chemical Formula 1], and then sequentially adding isopropyl alcohol and isopropyl ether to prepare the HCl salt of the compound represented by [Chemical Formula 1].

In another embodiment of the present disclosure, step (4) may be performed by adding methylene chloride (CH₂Cl₂) and methanol (MeOH) to the compound represented by [Chemical Formula 1] and performing cooling, and then adding hydrogen chloride (HCl) saturated in dioxane to prepare the HCl salt of the compound represented by [Chemical Formula 1].

In another embodiment of the present disclosure, step (4) may be performed by adding hydrogen chloride (HCl) gas to the compound represented by [Chemical Formula 1], and then adding ethyl ether to prepare the HCl salt of the compound represented by [Chemical Formula 1].

Furthermore, in order to achieve the above mentioned goals, the present disclosure provides a method for preparing a thiobenzimidazole derivative represented by [Chemical Formula 6], including the following steps:
(1) preparing a compound represented by [Chemical Formula 8] by adding methyl 3-mercaptopropanoate, Xantphos, DIEA (N,N-diisopropylethylamine), and Pd₂(dba)₃ to a compound represented by [Chemical Formula 7];
(2) preparing a compound represented by [Chemical Formula 9] by adding sodium ethoxide (EtONa) to the compound represented by [Chemical Formula 8] as prepared above;
(3) preparing a compound represented by [Chemical Formula 10] by adding one or more selected from the group consisting of 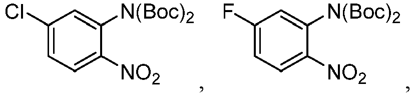 and 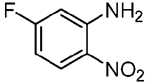 to the compound represented by [Chemical Formula 9] as prepared above; and
(4) preparing a compound represented by [Chemical Formula 6] by adding 1,3-bis(methoxycarbonyl)-2-methyl 2-thiopseudoeura to the compound represented by [Chemical Formula 10] as prepared above.

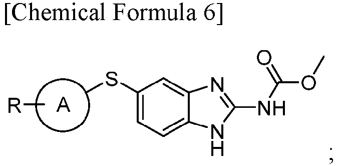

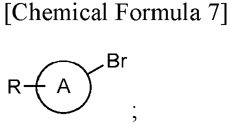

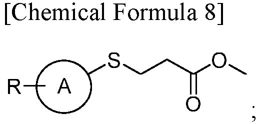

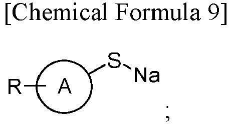

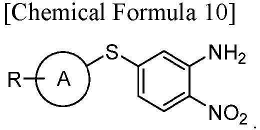

In an embodiment of the present disclosure, in Chemical Formulae 6 to 10, A may be a C₃-C₁₀ cyclic alkyl group, a heterocycloalkyl group, an aryl group, or a heteroaryl group, R may be one or more selected from the group consisting of hydrogen, a halogen group, a chained or cyclic alkyl group of C₁-C₁₀, a heterocycloalkyl group, an aryl group, and a heteroaryl group, the cyclic alkyl group, the heterocycloalkyl group, the aryl group, or the heteroaryl group may be unsubstituted or substituted with one or more selected from the group consisting of a C₁-C₁₀ chained alkyl group, an alkylsulfonyl group, and a carbonyl group, and the carbonyl group may be substituted with a chained or cyclic alkyl group of C₁-C₁₀, a heterocycloalkyl group, an aryl group, or a heteroaryl group.

In another embodiment of the present disclosure, A may be phenyl, furan, pyridine, pyrimidine, thiophen, thiazole, imidazole, pyrrole, triazole, etc., but is not limited thereto, and R may be hydrogen, a halogen group, a methyl group, an ethyl group, a propyl group, a piperazinyl group, a piperidinyl group, a morpholinyl group, a thiophenyl group, an imidazolyl group, a triazolyl group, 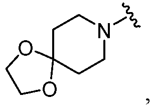 etc., but is not limited thereto. The piperazinyl or piperidinyl group may be unsubstituted or substituted with one or more selected from the group consisting of a methyl group, an ethyl group, a propyl group, a methylsulfone group, and 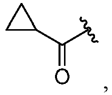 but is not limited thereto.

In another embodiment of the present disclosure, the compound represented by [Chemical Formula 6] may be any one selected from the group consisting of the following [Chemical Formula 6-1] to [Chemical Formula 6-6]: 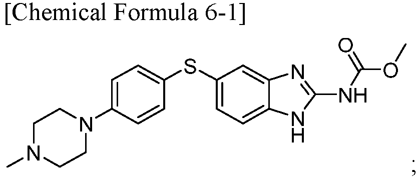 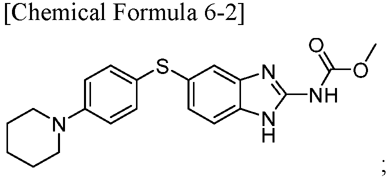 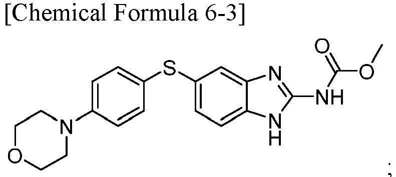 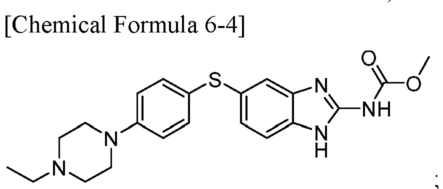 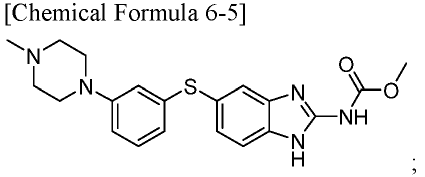 and

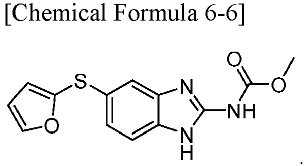

In another embodiment of the present disclosure, the compound represented by [Chemical Formula 7] may be any one selected from the group consisting of the following [Chemical Formula 7-1] to [Chemical Formula 7-6]:

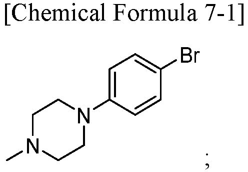

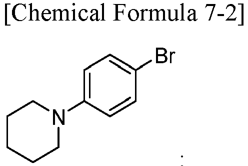

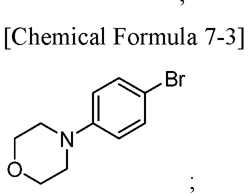

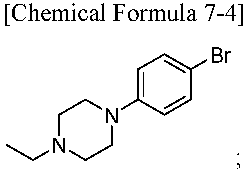

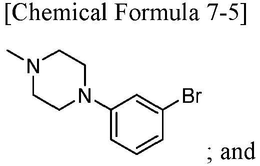

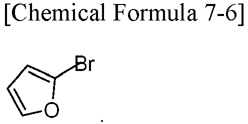

In another embodiment of the present disclosure, the compound represented by [Chemical Formula 8] may be any one selected from the group consisting of the following [Chemical Formula 8-1] to [Chemical Formula 8-6]: 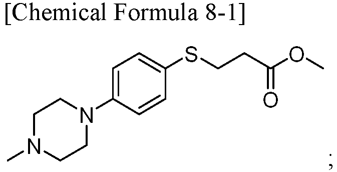 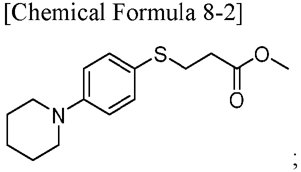 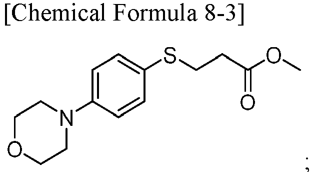 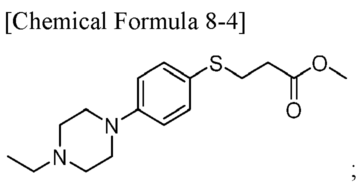 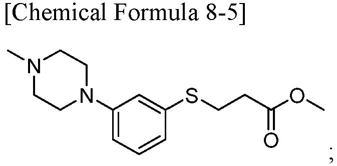 and

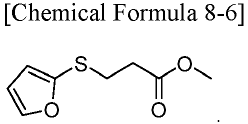

In another embodiment of the present disclosure, the compound represented by [Chemical Formula 9] may be any one selected from the group consisting of the following [Chemical Formula 9-1] to [Chemical Formula 9-6]: 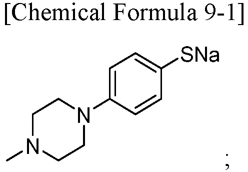 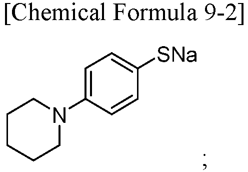 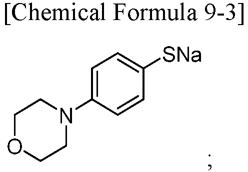 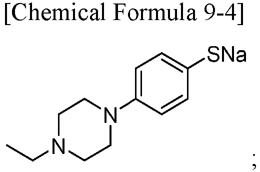 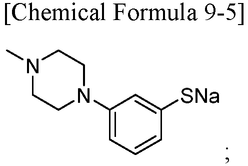 and

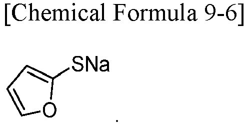

In another embodiment of the present disclosure, the compound represented by [Chemical Formula 10] may be any one selected from the group consisting of the following [Chemical Formula 10-1] to [Chemical Formula 10-6]: 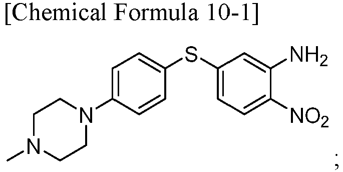 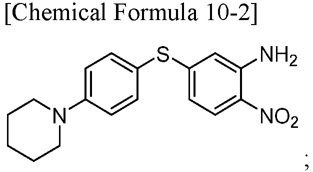 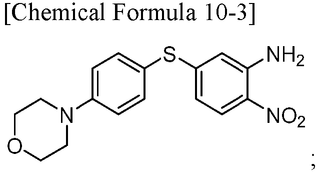 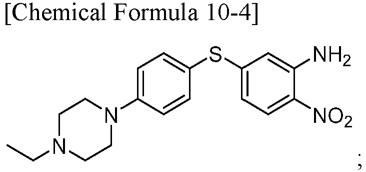 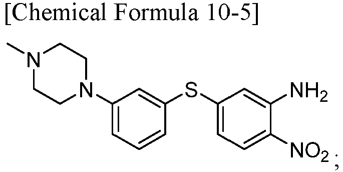 and

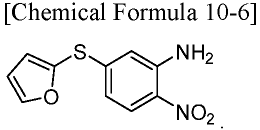

In another embodiment of the present disclosure, step (1) may be performed by sequentially adding methyl 3-mercaptopropanoate, Xantphos, DIEA (N,N-diisopropylethylamine), and Pd₂(dba)₃ to the compound represented by [Chemical Formula 7] in dioxane. After the addition is completed, stirring may be performed at 110°C for 16 hours.

In another embodiment of the present disclosure, step (2) may be performed by adding 20% sodium ethoxide (EtONa) to the compound represented by [Chemical Formula 8] in dry tetrahydrofuran (THF) at room temperature.

In another embodiment of the present disclosure, step (3) may be performed by adding K₂CO₃ along with one or more selected from the group consisting of 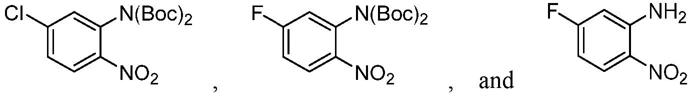 in 1-methyl-2-pyrrolidinone (NMP). After the addition is completed, stirring may be performed at 130°C for 16 hours.

In another embodiment of the present disclosure, step (4) may be performed by synthesizing [Chemical Formula 11] from the compound represented by [Chemical Formula 10] and then adding 1,3-bis(methoxycarbonyl)-2-methyl 2-thiopseudoeura in the presence of acetic acid (AcOH) to prepare the compound represented by [Chemical Formula 6]. After the addition is completed, stirring may be performed at 80°C for 2 hours.

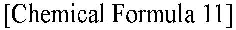

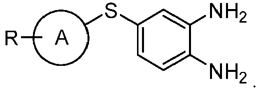

In an embodiment of the present disclosure, in Chemical Formulae 6 to 10, A may be a C₃-C₁₀ cyclic alkyl group, a heterocycloalkyl group, an aryl group, or a heteroaryl group, R may be one or more selected from the group consisting of hydrogen, a halogen group, a chained or cyclic alkyl group of C₁-C₁₀, a heterocycloalkyl group, an aryl group, and a heteroaryl group, the cyclic alkyl group, the heterocycloalkyl group, the aryl group, or the heteroaryl group may be unsubstituted or substituted with one or more selected from the group consisting of a C₁-C₁₀ chained alkyl group, an alkylsulfonyl group, and a carbonyl group, and the carbonyl group may be substituted with a chained or cyclic alkyl group of C₁-C₁₀, a heterocycloalkyl group, an aryl group, or a heteroaryl group.

In another embodiment of the present disclosure, the compound represented by [Chemical Formula 11] may be any one selected from the group consisting of the following [Chemical Formula 11-1] to [Chemical Formula 11-6]: 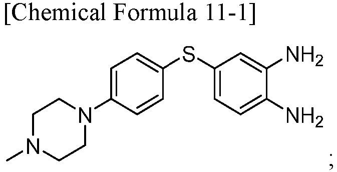 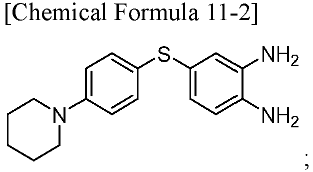 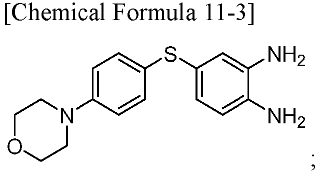 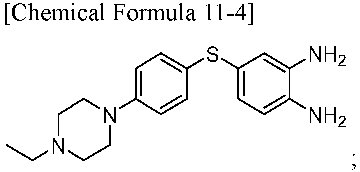 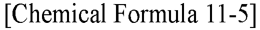 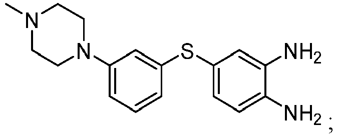 and

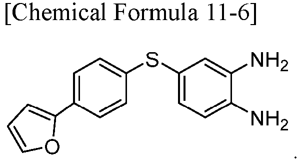

In another embodiment of the present disclosure, step (3) may be performed by adding methanol (MeOH), hydrogen gas (H₂), and palladium/carbon (Pd/C) or adding acetic acid (AcOH) and zinc (Zn) to the compound represented by [Chemical Formula 10] to synthesize the compound represented by [Chemical Formula 11].

In addition, the present disclosure provides a method for preparing a pharmaceutically acceptable salt of the thiobenzimidazole derivative represented by [Chemical Formula 6], including the following steps:
(1) preparing a compound represented by [Chemical Formula 8] by adding methyl 3-mercaptopropanoate, Xantphos, DIEA (N,N-diisopropylethylamine), and Pd₂(dba)₃ to a compound represented by [Chemical Formula 7];
(2) preparing a compound represented by [Chemical Formula 9] by adding sodium ethoxide (EtONa) to the compound represented by [Chemical Formula 8] as prepared above;
(3) preparing a compound represented by [Chemical Formula 10] by adding one or more selected from the group consisting of 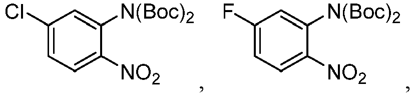 and 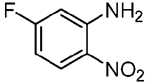 to the compound represented by [Chemical Formula 9] as prepared above;
(4) preparing a compound represented by [Chemical Formula 6] by adding 1,3-bis(methoxycarbonyl)-2-methyl 2-thiopseudoeura to the compound represented by [Chemical Formula 10] as prepared above; and
(5) preparing a hydrogen chloride (HCl) salt of the compound represented by [Formula 6] by adding HCl to the compound represented by [Formula 6] as prepared above.

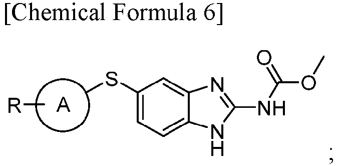

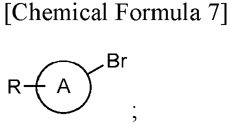

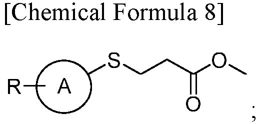

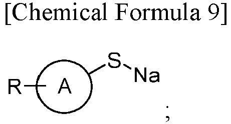

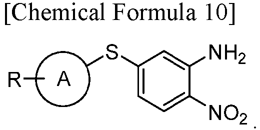

In an embodiment of the present disclosure, in Chemical Formulae 6 to 10, A may be a C₃-C₁₀ cyclic alkyl group, a heterocycloalkyl group, an aryl group, or a heteroaryl group, R may be one or more selected from the group consisting of hydrogen, a halogen group, a chained or cyclic alkyl group of C₁-C₁₀, a heterocycloalkyl group, an aryl group, and a heteroaryl group, the cyclic alkyl group, the heterocycloalkyl group, the aryl group, or the heteroaryl group may be unsubstituted or substituted with one or more selected from the group consisting of a C₁-C₁₀ chained alkyl group, an alkylsulfonyl group, and a carbonyl group, and the carbonyl group may be substituted with a chained or cyclic alkyl group of C₁-C₁₀, a heterocycloalkyl group, an aryl group, or a heteroaryl group.

In another embodiment of the present disclosure, step (5) may be performed by adding methanol (MeOH) and hydrogen chloride (HCl) to the compound represented by [Chemical Formula 6], and then sequentially adding isopropyl alcohol and isopropyl ether to prepare the HCl salt of the compound represented by [Chemical Formula 6].

In another embodiment of the present disclosure, step (5) may be performed by adding methylene chloride (CH₂Cl₂) and methanol (MeOH) to the compound represented by [Chemical Formula 6] and performing cooling, and then adding hydrogen chloride (HCl) saturated in dioxane to prepare the HCl salt of the compound represented by [Chemical Formula 6].

In another embodiment of the present disclosure, step (5) may be performed by adding hydrogen chloride (HCl) gas to the compound represented by [Chemical Formula 6], and then adding ethyl ether to prepare the HCl salt of the compound represented by [Chemical Formula 6].

### EFFECTS OF THE INVENTION

The present disclosure relates to a method for preparing a thiobenzimidazole derivative and a novel intermediate therefor, and the present disclosure may efficiently synthesize a thiobenzimidazole derivative represented by [Chemical Formula 1] having anti-cancer activity by using an intermediate containing O-ethylcarbonodithioate. In addition, the present disclosure may efficiently synthesize a thiobenzimidazole derivative represented by [Chemical Formula 1] having anti-cancer activity by using an intermediate containing methyl 3-mercaptopropanoate.

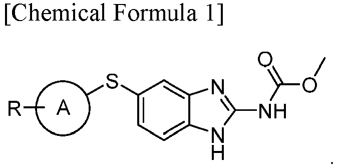

Furthermore, the thiobenzimidazole derivative represented by [Chemical Formula 1] synthesized by the preparation method of the present disclosure is activated in cancer cells to inhibit tubulin polymerization, and to induce apoptosis when administered to an individual, thereby exhibiting cytotoxicity, and thus may be utilized for preventing or treating cancer.

The effects of the present disclosure are not limited to those mentioned above, and other effects not mentioned may be clearly understood by one of ordinary skill in the art from the following descriptions.

### BEST MODE FOR CARRYING OUT THE INVENTION

The present disclosure provides a method for preparing a thiobenzimidazole derivative or a pharmaceutically acceptable salt thereof via a novel intermediate.

The present disclosure provides a method for preparing a thiobenzimidazole derivative, including the following steps:
(1) preparing a compound represented by [Chemical Formula 3] by adding potassium O-ethylcarbonodithioate to a compound represented by [Chemical Formula 2];
(2) preparing a compound represented by [Chemical Formula 4] by adding 5-chloro-2-nitroaniline to the compound represented by [Chemical Formula 3] as prepared above; and
(3) preparing a compound represented by [Chemical Formula 1] by adding 1,3-bis(methoxycarbonyl)-2-methyl 2-thiopseudoeura to the compound represented by [Chemical Formula 4] as prepared above.

In addition, there is provided a method for preparing a pharmaceutically acceptable salt of a thiobenzimidazole derivative, the method including the step of preparing a HCl salt of a compound represented by [Chemical Formula 1] by adding methanol (MeOH) and chlorine to the compound represented by [Chemical Formula 1] after performing step (3).

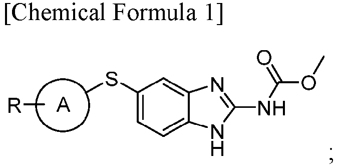

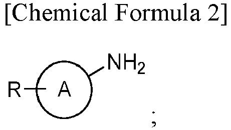

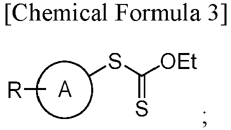

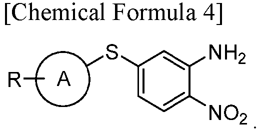

In an embodiment of the present disclosure, in Chemical Formulae 1 to 4, A may be a C₃-C₁₀ cyclic alkyl group, a heterocycloalkyl group, an aryl group, or a heteroaryl group, R may be one or more selected from the group consisting of hydrogen, a halogen group, a chained or cyclic alkyl group of C₁-C₁₀, a heterocycloalkyl group, an aryl group, and a heteroaryl group, the cyclic alkyl group, the heterocycloalkyl group, the aryl group, or the heteroaryl group may be unsubstituted or substituted with one or more selected from the group consisting of a C₁-C₁₀ chained alkyl group, an alkylsulfonyl group, and a carbonyl group, and the carbonyl group may be substituted with a chained or cyclic alkyl group of C₁-C₁₀, a heterocycloalkyl group, an aryl group, or a heteroaryl group.

As used herein, the term "substitution" refers to a reaction in which an atom or atomic group contained in a molecule of a compound is replaced with another atom or atomic group.

As used herein, the term "chained" refers to a molecule with a chain structure, and the chained structure is a chemical structure in which carbon atoms are connected in a chain shape, and there are two types: straight chain and branched chain.

As used herein, the term "cyclic" refers to a structure in which the backbone of an organic compound has two ends that are connected to form a ring.

As used herein, the term "chained or cyclic alkyl group" means a monovalent linear or branched or cyclic saturated hydrocarbon residue having 1 to 20 carbon atoms and consisting only of carbon and hydrogen atoms. Examples of such alkyl groups include, but are not limited to, methyl, ethyl, propyl, isopropyl, butyl, isobutyl, 2-butyl, 3-butyl, pentyl, n-hexyl, cyclobutyl, cyclopentyl, cyclohexyl, etc.

As used herein, the term "heterocycloalkyl group" typically refers to a saturated or unsaturated (but not aromatic) cyclohydrocarbon, which may be optionally unsubstituted, mono-substituted or poly-substituted, wherein at least one of the heteroatoms in the structure is selected from N, O or S.

As used herein, the term "aryl group" means an unsaturated aromatic ring compound having 3 to 12 carbon atoms having a single ring (e.g., phenyl) or a plurality of condensed rings (e.g., naphthyl). Examples of such aryl groups include, but are not limited to, phenyl, naphthyl, etc.

As used herein, the term "heteroaryl group" refers to a single ring or a plurality of condensed rings in which at least one of the atoms constituting the ring is a heteroatom of N, O, or S. Examples of such heteroaryl groups include, but are not limited to, pyridyl groups, pyrimidinyl groups, pyrazinyl groups, oxazolyl groups, furyl groups, etc.

As used herein, the "halogen group" may be fluorine (F), chloride (Cl), bromine (Br), or iodine (I), etc.

As used herein, the term "pharmaceutically acceptable salt" means a formulation of a compound that does not cause serious irritation to the organism to which the compound is administered and does not impair the biological activity and properties of the compound. The pharmaceutically acceptable salt of the compound of the present disclosure may be obtained by reacting the compound of the present disclosure with inorganic acids such as hydrochloric acid, hydrobromic acid, sulfuric acid, nitric acid, and phosphoric acid, sulfonic acids such as methanesulfonic acid, ethanesulfonic acid, and p-toluenesulfonic acid, organic carboxylic acids such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid, and salicylic acid. Also, the pharmaceutically acceptable salt of the compound of the present disclosure may be obtained by reacting the compound of the present disclosure with a base to form salts such as ammonium salt, alkali metal salt such as sodium or potassium salt, alkaline earth metal salt such as calcium or magnesium salt, salts of organic bases such as dicyclohexylamine, N-methyl-D-glucamine, tris(hydroxymethyl)methylamine and salts of amino acids such as arginine and lysine.

Furthermore, the thiobenzimidazole derivative or the pharmaceutically acceptable salt thereof may include not only pharmaceutically acceptable salts, but also all salts, hydrates and solvates that may be prepared by conventional methods.

Also, the present disclosure provides a method for preparing a thiobenzimidazole derivative or a pharmaceutically acceptable salt thereof via a novel intermediate.

There is provided a method for preparing the thiobenzimidazole derivative, including the following steps:
(1) preparing a compound represented by [Chemical Formula 8] by adding methyl 3-mercaptopropanoate, Xantphos, DIEA (N,N-diisopropylethylamine), and Pd₂(dba)₃ to the compound represented by [Chemical Formula 7];
(2) preparing a compound represented by [Chemical Formula 9] by adding sodium ethoxide (EtONa) to the compound represented by [Chemical Formula 8] as prepared above;
(3) preparing a compound represented by [Chemical Formula 10] by adding one or more selected from the group consisting of 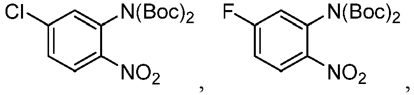 and 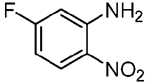 to the compound represented by [Chemical Formula 9] as prepared above; and
(4) preparing a compound represented by [Chemical Formula 6] by adding 1,3-bis(methoxycarbonyl)-2-methyl 2-thiopseudoeura to the compound represented by [Chemical Formula 10] as prepared above.

In addition, there is provided a method for preparing a pharmaceutically acceptable salt of a thiobenzimidazole derivative, the method including the step of preparing a HCl salt of a compound represented by [Chemical Formula 6] by adding methanol (MeOH) and chlorine to the compound represented by [Chemical Formula 6] after performing step (4).

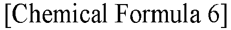

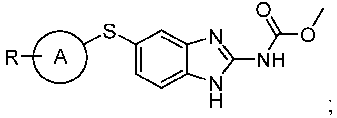

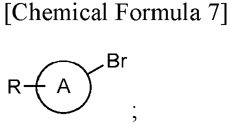

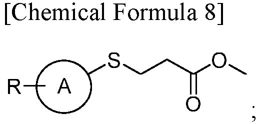

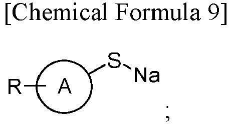

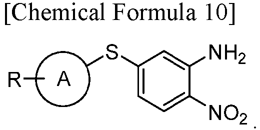

In an embodiment of the present disclosure, in Chemical Formulae 6 to 10, A may be a C₃-C₁₀ cyclic alkyl group, a heterocycloalkyl group, an aryl group, or a heteroaryl group, R may be one or more selected from the group consisting of hydrogen, a halogen group, a chained or cyclic alkyl group of C₁-C₁₀, a heterocycloalkyl group, an aryl group, and a heteroaryl group, the cyclic alkyl group, the heterocycloalkyl group, the aryl group, or the heteroaryl group may be unsubstituted or substituted with one or more selected from the group consisting of a C₁-C₁₀ chained alkyl group, an alkylsulfonyl group, and a carbonyl group, and the carbonyl group may be substituted with a chained or cyclic alkyl group of C₁-C₁₀, a heterocycloalkyl group, an aryl group, or a heteroaryl group.

Furthermore, the thiobenzimidazole derivative or the pharmaceutically acceptable salt thereof may include not only pharmaceutically acceptable salts, but also all salts, hydrates and solvates that may be prepared by conventional methods.

The terminology used in embodiments is for the purpose of describing particular embodiments only and is not intended to be limiting. The singular forms "a," "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, components, and/or groups thereof but do not preclude the presence or addition of one or more other features, integers, steps, operations, elements, components, and/or groups thereof.

Unless otherwise defined, all terms including technical or scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

As the inventive concept allows for various changes and numerous embodiments, particular embodiments will be illustrated in the drawings and described in detail in the written description. However, such illustrations and descriptions are not intended to limit the present disclosure to specific embodiments, and should be understood as including all changes, equivalents, and replacements within the idea and the technical scope of the present disclosure. In the description of embodiments, detailed description of well-known related structures or functions will be omitted when it is deemed that such description will cause ambiguous interpretation of the present disclosure.

Hereinafter, embodiments will be described in detail with reference to the accompanying drawings. However, since various changes may be made to the embodiments, it will be understood that the descriptions of the embodiments do not limit or otherwise restrict the scope of the patent application. With respect to the embodiments, it should be understood that the scope of the rights shall include all modifications, equivalents, and replacements.

### MODE FOR CARRYING OUT THE INVENTION

### Example 1. Synthesis of Chemical formula 1-1

The synthesis process of the thiobenzimidazole derivative of Chemical Formula 1-1 is shown by Reaction Scheme 1 presented below.

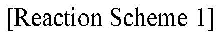

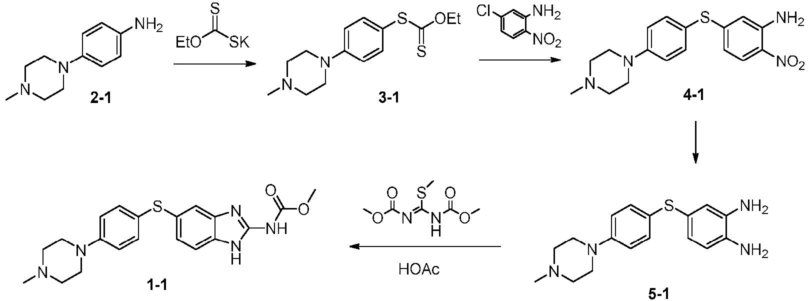

### 1.1. Synthesis of 4-(4-methylpiperazin-1-yl) aniline (Chemical Formula 2-1)

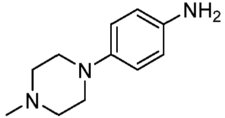

To a solution of 4-Fluoronitrobenzene (2.82 g, 0.02 mmol) in DMF (30 mL) were sequentially added methylpiperazine (2.64 g, 1.2 eq) and potassium carbonate (4.14 g, 1.5 eq), and the reaction mixture was reacted at 80-100°C for 20 h. The reaction mixture was cooled to room temperature, and then ice water (100 mL) was added to form a solid precipitate, which was filtered to obtain 4.1 g of 1-methyl-4-(4-nitrophenyl)piperazine as a light brown solid (94% yield).

To a solution of 1-methyl-4-(4-nitrophenyl)piperazine (3.6 g, 16.29 mmol) in methanol (100 mL) was added Pd/C (5 eq) and the reaction mixture was reacted in a Parr reactor for 15 h. The solvent was removed by filtration to afford 3.0 g of 4-(4-methylpiperazin-1-yl)aniline (Chemical Formula **2-1**) as a yellow solid (96% yield).

### 1.2. Synthesis of O-ethyl S-(4-(4-methylpiperazin-1-yl)phenyl)carbonodithioate (Chemical Formula 3-1)

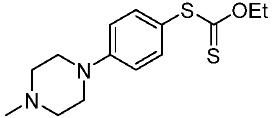

### 1.2.1. Method 1

To a solution of 4-(4-methylpiperazin-1-yl)aniline (Chemical Formula **2-1,** 1.95 g, 10.2 mmol) in THF/H₂O (40 mL/40 mL), was added a concentrated solution of H₂SO₄ (1.70 g, 17.3 mmol). Then, to the mixture was slowly added sodium nitrite (NaNO₂) (1.40 g, 20.4 mmol) dissolved in water (3 mL) and the mixture was stirred at -5 to 0°C for 2 h. To the mixture was then added potassium O-ethylcarbonodithioate (8.16 g, 51 mmol) and the mixture was stirred at room temperature for 2 h. After the reaction was completed, the mixture was poured into water (100 mL) and extracted with EA (100 mL×2). The organic layer was dried over Na₂SO₄, concentrated and purified by reverse column chromatography (ACN/H₂O) to obtain 2.30 g of O-ethyl S-(4-(4-methylpiperazin-1-yl)phenyl)carbonodithioate (Chemical Formula 3-1) as a brown oil (76% yield).
¹H-NMR (CDCl₃, 400 MHz) δ 7.37(d, J=8.8Hz, 2H), 6.94(d, J=8.8Hz, 2H), 4.64(q, 2H), 3.33(m, 4H), 2.59(m, 4H), 2.37(s, 3H), 1.36(t, 3H)
MS Calcd.: 296; MS Found: 297 [M+H]⁺.

### 1.2.2. Method 2

An aqueous solution of 4-(4-methylpiperazin-1-yl)aniline (Chemical Formula **2-1**, 3.0 g, 15.7 mmol) in distilled water (45 mL) was cooled to 0°C in an ice bath. To the solution was slowly added dropwise, c-HCl (2.4 mL) and then a solution of previously prepared sodium nitrate (NaNO₃, 1.3 g, 1.2 eq) in distilled water (10 mL), over 30 min. Under the same conditions, a solution of previously prepared potassium O-ethylcarbonodithioate (3.77 g, 1.5 eq) in distilled water (10 mL) was slowly added, and when stirring did not work well, the reaction temperature was raised to 50°C and then the solution was added. The reaction mixture was stirred for 1 hour, then extracted with ethyl acetate and chloroform solvent, dried and filtered. The resulting reaction mixture was separated by column chromatography under appropriate conditions with ethyl acetate and methanol (9:1, v/v), and methylene chloride and methanol (9:1. v/v) as eluents to afford 1.72 g of O-ethyl S-(4-(4-methylpiperazin-1-yl)phenyl)carbonodithioate (Chemical Formula **3-1**) as a light brown solid with an R_{f} value of 0.2 (37% yield).

### 1.3. Synthesis of 5-((4-(4-Methylpiperazin-1-yl)phenyl)thio)-2-nitroaniline (Chemical Formula 4-1)

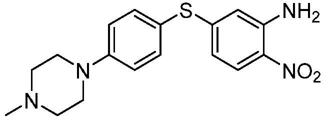

### 1.3.1. Method 1

To a solution of O-ethyl S-(4-(4-methylpiperazin-1-yl)phenyl)carbonodithioate (Chemical Formula **3-1,** 1.00 g, 3.38 mmol) in THF/MeOH (20 mL/10 mL), were added 5-chloro-2-nitroaniline (1.16 g, 6.76 mmol) and CH₃ONa (5 M in MeOH, 2.0 mL, 10.14 mmol) and the mixture was stirred at 55°C for 16 h. After the reaction was completed, the pH of the mixture was adjusted to pH = 7-8 with AcOH, the mixture was concentrated, and then the mixture was purified by silica gel column chromatography (PE/EA = 5:1, then DCM / MeOH = 10:1) to obtain 966 mg of 5-((4-(4-methylpiperazin-1-yl)phenyl)thio)-2-nitroaniline (Chemical Formula **4-1**) as an orange solid (yield 69%).
¹H-NMR (CDCl₃, 400 MHz) δ 7.86(d, J=8.8Hz, 1H), 7.45(s, 1H), 7.39(d, J=8.8Hz, 2H), 7.06(d, J=8.8Hz, 2H), 6.52(s, 1H), 6.27(d, J=9.2Hz, 1H), 3.26(m, 4H), 2.45(m, 4H), 2.23(s, 3H)
MS Calcd.: 344; MS Found: 345 [M+H]⁺.

### 1.3.2. Method 2

In a solution of O-ethyl S-4-(4-methylpiperazin-1-yl)phenyl)carbonothioate (Chemical Formula 3-1, 0.89 g, 3.0 mmol) in THF (30 mL), a mixed solution of previously prepared 5-chloro-2-nitroaniline (0.52 g, 3.0 mmol) in distilled water (10 mL) and methanol (50 mL) was reacted at 65°C for 16 h. The reaction mixture was cooled to room temperature and then extracted with an organic solvent while a pH of 7-8 was maintained. The resulting reaction mixture was separated by column chromatography under appropriate conditions using a solvent of methylene chloride and methanol (9: 1. v/v) to obtain 0.67 g of 5-((4-(4-methylpiperazin-1-yl)phenyl)thio)-2-nitroaniline (Chemical Formula **4-1**) as a light brown solid with an R_{f} value of 0.15 (65% yield).

### 1.4. Synthesis of methyl (5-((4-(4-methylpiperazin-1-yl)phenyl)thio)-1H-benzo[d]imidazol-2-yl)carbamate (Chemical Formula 1-1)

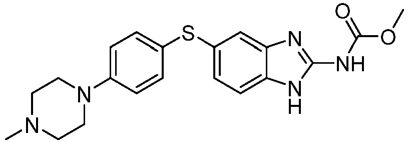

### 1.4.1. Method 1

To a solution of 5-((4-(4-methylpiperazin-1-yl)phenyl)thio)-2-nitroaniline (Chemical Formula **4-1,** 300 mg, 0.87 mmol) in AcOH (15 M) were added 1,3-bis(methoxycarbonyl)-2-methyl-2-thiopseudoeura (358 mg, 1.74 mmol) and Zn (452 mg, 6.96 mmol), and the mixture was stirred at 75°C for 2 h. After the reaction was completed, the solvent was removed. The mixture was dissolved in MeOH (20 mL), filtered, and the filtrate was concentrated and purified by prep-HPLC to obtain 0.110 g of methyl (5-((4-(4-methylpiperazin-1-yl)phenyl)thio)-1H-benzo[d]imidazol-2-yl)carbamate (Chemical Formula **1-1**) as a pale white solid (yield 32%).

¹H-NMR (400 MHz, DMSO-d₆): δ 11.61 (s, 2H), 7.34 (d, 1H, J = 8.0Hz), 7.29 (s, 1H), 7.21 (d, 2H, J = 8.8 Hz), 7.02 (d, 1H, J = 8.0 Hz), 6.92(d, 2H, J = 8.8 Hz), 3.74 (s, 3H), 3.16-3.10 (m, 4H), 2.44-2.40 (m, 4H), 2.20(s, 3H).

LCMS [mobile phase: 85% water (0.1% TFA) and 15% CH₃CN - 50% water (0.1% TFA) and 50% CH₃CN for 6 min, and finally 0.5 min under these conditions], purity 99 %, Rt= 2.741 min; MS Calcd.: 397; MS Found: 398 [M+H]⁺.

### 1.4.2. Method 2

### (1) Synthesis of 4-((4-(4-methylpiperazin-1-yl)phenyl)thio)benzene-1,2-diamine (Chemical Formula 5-1)

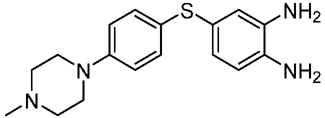

### 1) Method 2-1

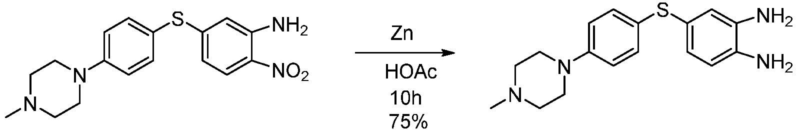

5-((4-(4-methylpiperazin-1-yl)phenyl)thio)-2-nitroaniline (Chemical Formula **4-1,** 0.600 g, 1.74 mmol) was dissolved in acetic acid (8 mL). To the mixture was added Zn powder (0.5 g). Then, the reaction mixture was stirred at room temperature for 10 h to obtain 0.410 g of 4-((4-(4-methylpiperazin-1-yl)phenyl)thio)benzene-1,2-diamine (Chemical Formula 5-1) as a light brown solid (yield 75%).

¹H NMR (CDCl₃, 400 MHz) δ 7.03 (d, J=8.8 Hz, 2H), 6.84 (d, J=8.8 Hz, 2H), 6.56 (s, 1H), 6.46 (s, 2H), 4.57-4.66 (m,4H), 3.06-3.08 (m, 4H), 2.40-2.42 (m, 4H), 2.19 (s, 1H),

### 2) Method 2-2

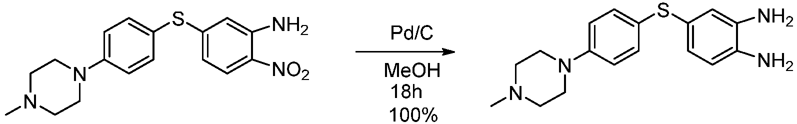

To a solution of 5-((4-(4-methylpiperazin-1-yl)phenyl)thio)-2-nitroaniline (Chemical Formula **4-1,** 1248 mg, 3.624 mmol) in MeOH (40 mL) were sequentially added a solution of MeOH/NH₃ (12 mL) and Pd/C (360 mg), and the mixture was stirred in the presence of H₂ gas at room temperature for 18 h. The reaction mixture was filtered and the filtrate was concentrated to obtain 1.140 g of 4-((4-(4-methylpiperazin-1-yl)phenyl)thio)benzene-1,2-diamine (Chemical Formula **5-1**) as a light brown solid (yield 100%).

### 3) Method 2-3

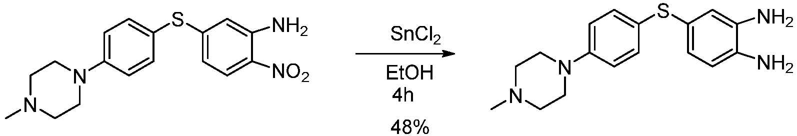

To a solution of 5-((4-(4-methylpiperazin-1-yl)phenyl)thio)-2-nitroaniline (Chemical Formula **4-1,** 0.344 g, 1.0 mmol) in ethanol (10 mL) was added tin chloride two hydrate (6 eq.). The mixture was heated and refluxed for 4 h, then cooled to room temperature. Water (50 mL) was added. Extraction was performed with excess ethyl acetate solvent, then the solvent was removed by evaporation under reduced pressure to obtain a solid phase reaction mixture. The mixture was separated and purified by column chromatography on silica gel using a solvent of ethyl acetate and hexane (3:1, v/v) to obtain 0.151 g of 4-((4-(4-methylpiperazin-1-yl)phenyl)thio)benzene-1,2-diamine (Chemical Formula **5-1**) as a light brown solid (yield 48%).

### (2) Synthesis of methyl (5-((4-(4-methylpiperazin-1-yl)phenyl)thio)-1H-benzo[d]imidazol-2-yl)carbamate

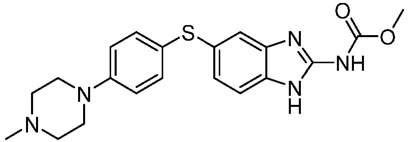

4-((4-(4-methylpiperazin-1-yl)phenyl)thio)benzene-1,2-diamine (Chemical Formula **5-1,** 0.4 g, 1.27 mmol) and 1,3-bis(methoxycarbonyl)-2-methyl-2-thiopseudoeura (1.5 g, 2.5 eq) were dissolved in a 5% acetic acid ethanol solution (4 mL) and the mixture was reacted by heating and refluxing for 5 h. To the reaction mixture was added Methanol (20 mL). Then the reaction mixture was cooled with cold water to obtain a solid product and filtered. The filtrate was washed with methanol, urea byproducts were removed, and then the filtrate was dried to obtain 0.4 g of methyl (5-((4-(4-methylpiperazin-1-yl)phenyl)thio)-1H-benzo[d]imidazol-2-yl)carbamate (Chemical Formula **1-1**) as a white solid (79% yield).

### 1.5. Synthesis of Methyl(5-((4-(4-methylpiperazin-1-yl)phenyl)thio)-1H-benzo[d]imidazol-2-yl)carbamate hydrochloric acid salt

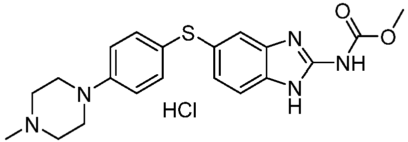

### 1.5.1. Method 1

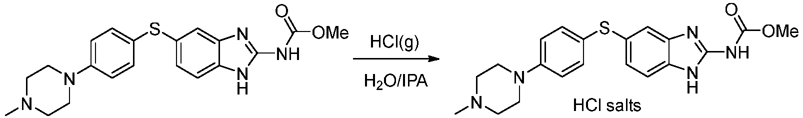

To a solution of methyl (5-((4-(4-methylpiperazin-1-yl)phenyl)thio)-1H-benzo[d]imidazol-2-yl)carbamate (Chemical Formula **1-1,** 370 mg, 0.93 mmol) in methanol (75 mL) was injected hydrogen chloride (HCl) gas at 2-minute intervals until the pH reached 1 or less. The reactant was evaporated under reduced pressure to obtain an oil-form product, and then isopropyl alcohol (37 mL) was added again. The mixture was dissolved by heating, and then cooled to room temperature to produce a solid suspension. Isopropyl ether (35 mL) was added to the solid suspension in portions, then the suspension was stirred for 3 h, and then filtered. The filtrate was washed with a mixed solvent of IPA and IPE, dried with hot air at 50°C for 1 hour, and vacuum dried at 40°C for 2 h to obtain 0.350 g of methyl (5-((4-(4-methylpiperazin-1-yl)phenyl)thio)-1H-benzo[d]imidazol-2-yl)carbamate hydrochloric acid salt as a off-white solid (87% yield).

¹H NMR (D₂O, 400 MHz) δ 2.93(s, 3H), 3.08-3.14 & 3.19-3.25 (2m,4H), 3.61(d, J = 12.5 Hz, 2H), 3.64(d, J = 12.5 Hz, 2H), 3.89(s, 3H), 7.04-7.44(m, 7H).

### 1.5.2. Method 2

A mixed solution of methyl (5-((4-(4-methylpiperazin-1-yl)phenyl)thio)-1H-benzo[d]imidazol-2-yl)carbamate (Chemical Formula **1-1,** 500 mg, 1.257 mmol) in methylene chloride (CH₂Cl₂, 30 mL) and methanol (30 mL) was cooled in an ice bath. To the mixture was added 4 moles of hydrochloric acid solution (HCl, 5.03 mmol, 4 eq.) saturated in dioxane, then the mixture was stirred for 1 hour. After the reaction was completed, the solvent was removed by evaporation under reduced pressure and the residue was washed with acetone to obtain methyl (5-((4-(4-methylpiperazin-1-yl)phenyl)thio)-1H-benzo[d]imidazol-2-yl)carbamate hydrochloric acid salt (500 mg) as an off-white solid (yield 91%).

### 1.5.3. Method 3

Sodium chloride (NaCl) was added to a round-bottom flask. Concentrated sulfuric acid (c-H₂SO₄) was added dropwise. The resulting hydrogen chloride gas was injected through a tube into a suspension of methyl (5-((4-(4-methylpiperazin-1-yl)phenyl)thio)-1H-benzo[d]imidazol-2-yl)carbamate (Chemical Formula 1-1, 40 mg, 0.1 mmol) in methanol (10 mL), creating an acidic solution. A precipitate was formed. Part of the solvent was removed, and to the residue was added ethyl ether and the residue was filtered to obtain methyl (5-((4-(4-methylpiperazin-1-yl)phenyl)thio)-1H-benzo[d]imidazol-2-yl)carbamate hydrochloric acid salt (30 mg) as a gray product (yield 69%).

### Example 2. Synthesis of Formula 1-2

The synthesis process of the thiobenzimidazole derivative of Chemical Formula 1-2 is shown by Reaction Scheme 2 presented below.

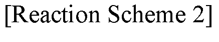

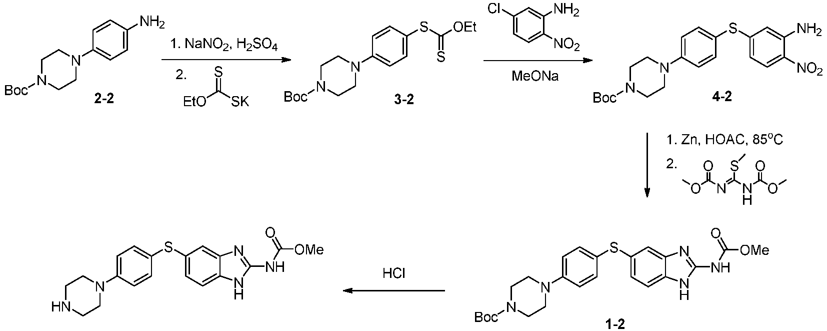

### 2.1. Synthesis of tert-butyl 4-(4-((ethoxycarbonothioyl)thio)phenyl)piperazine-1-carboxylate (Chemical Formula 3-2)

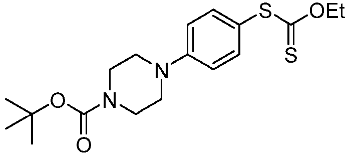

To a solution of tert-butyl 4-(4-aminophenyl)piperazine-1-carboxylate (Chemical Formula 2-2, 2.77 g, 10.2 mmol) in THF/H₂O (40 mL/40 mL) was added a concentrated solution of H₂SO₄ (1.96 g, 20.0 mmol). Then, NaNO₂ (2.10 g, 30 mmol) dissolved in water (3.0 mL) was slowly added and the mixture was stirred at -5°C to 0°C for 2 h. Potassium O-ethyl carbonodithioate (9.60 g, 60 mmol) was added and the mixture was stirred at room temperature for 2 h. After the reaction was completed, the mixture was poured into water (100 mL) and the mixture was extracted with EA (100 mL×3). The organic layer was dried over Na₂SO₄, concentrated, and purified by reverse column chromatography (ACN/H₂O) to obtain 2.00 g of tert-butyl 4-(4-((ethoxycarbonothioyl)thio)phenyl)piperazine-1-carboxylate (Chemical Formula **3-2**) as an orange solid (yield 52%).

### 2.2. Synthesis of tert-butyl 4-(4-((3-amino-4-nitrophenyl)thio)phenyl)piperazine-1-carboxylate (Chemical Formula 4-2)

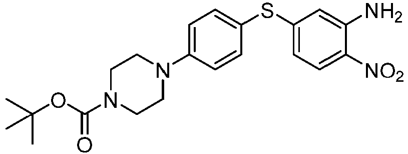

To a solution of tert-butyl 4-(4-((ethoxycarbonothioyl)thio)phenyl)piperazine-1-carboxylate (Chemical Formula **3-2,** 1.15 g, 3.00 mmol) in THF/MeOH/H₂O (30 mL/50 mL/10 mL), were added 5-chloro-2-nitroaniline (520 mg, 3.00 mmol) and NaOH (480 mg, 12 mmol). The mixture was stirred at 65°C for 16 h. After the reaction was completed, the pH of the mixture was adjusted to pH = 7-8 with AcOH, then the mixture was concentrated and purified by reverse column chromatography (ACN/H₂O) to obtain 600 mg of tert-butyl 4-(4-((3-amino- 4-nitrophenyl)thio)phenyl)piperazine-1-carboxylate (Chemical Formula **4-2**) as a brown solid (yield 46%).

### 2.3. Synthesis of tert-butyl 4-(4-((2-((methoxycarbonyl)amino)-1H-benzo[d]imidazol-6-yl)thio)phenyl)piperazine-1-carboxylate (Chemical Formula 1-2)

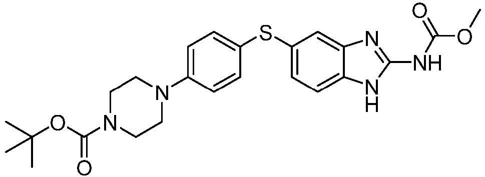

To a solution of tert-butyl 4-(4-((3-amino-4-nitrophenyl)thio)phenyl)piperazine-1-carboxylate (Chemical Formula **4-2,** 500 mg, 1.16 mmol) in AcOH (30 mL) were added 1,3-bis(methoxycarbonyl)-2-methyl-2-thiopseudoeura (478 mg, 2.32 mmol) and Zn powder (603 mg, 9.28 mmol). The mixture was stirred at 75°C for 20 h. After the reaction was completed, the solvent was removed. The mixture was dissolved in MeOH (50 mL), filtered, and the filtrate was concentrated and purified by reverse column chromatography (ACN/H₂O) to obtain 0.300 g of tert-butyl 4-(4-((2-((methoxycarbonyl)amino)-1H-benzo[d]imidazol-6-yl)thio)phenyl)piperazine-1-carboxylate (Chemical Formula **1-2**) as a yellow solid (yield 53%).

### 2.4. Synthesis of methyl (6-((4-(piperazin-1-yl)phenyl)thio)-1H-benzo[d]imidazol-2-yl)carbamate

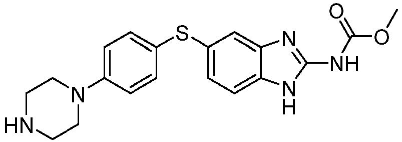

To a solution of tert-butyl 4-(4-((2-((methoxycarbonyl)amino)-1H-benzo[d]imidazol-6-yl)thio)phenyl)piperazine-1-carboxylate (Chemical Formula **1-2,** 300 mg, 0.62 mmol) in dioxane (6 mL) was added HCl/dioxane (6 mL, 6 mol/L), and the mixture was stirred at room temperature for 2 h. After the reaction was completed, the solvent was removed and Et₃N was added. The mixture was concentrated and purified by prep-HPLC to obtain 0.100 g of methyl (6-((4-(piperazin-1-yl)phenyl)thio)-1H-benzo[d]imidazol-2-yl)carbamate as an off-white solid (yield 42%).
¹H-NMR (400 MHz, DMSO-d₆): δ 7.33 (d, 1H, *J = 8.0* Hz), 7.29 (s, 1H), 7.21 (d, 1H, *J = 8.8* HZ), 7.03-7.00 (m, 1H), 6.90 (d, 2H, *J* = 8.8 Hz), 3.74 (s, 3H), 3.06-3.03 (m, 4H), 2.82-2.79 (m, 4H)

### Example 3. Synthesis of Formula 1-3

The synthesis process of the thiobenzimidazole derivative of Chemical Formula 1-3 is shown by Reaction Scheme 3 presented below.

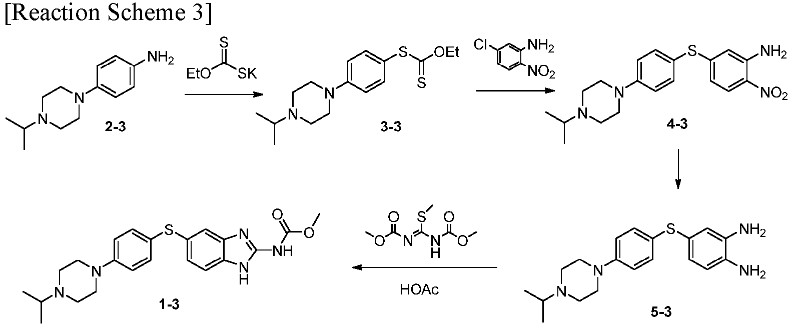

### 3.1. Synthesis of O-ethyl S-(4-(4-isopropylpiperazin-1-yl)phenyl)carbonodithioate (Chemical Formula 3-3)

To a mixed solution of 4-[4-(1-Methylethyl)-1-piperazinyl]benzenamine (Chemical Formula **2-3,** 2.19 g, 10 mmol) in THF/H₂O (50 mL/50 mL) were slowly sequentially added dropwise sulfuric acid (1.7 g, 17.3 mmol) and a solution of NaNO₂ (1.4 g, 20.4 mmol) dissolved in water (3 mL) in an ice bath at 0-5°C. The reaction mixture was stirred for 30 min, potassium O-ethyl carbonodithioate (8.18 g, 51 mmol) was added, and the reaction mixture was stirred at room temperature for 2 h. After the reaction was completed, the resulting mixture was poured into water (100 mL) and extracted with ethyl acetate. The organic layer was dried over anhydrous sodium sulfate, the solvent was concentrated by evaporation under reduced pressure, and then the residue was separated and purified by a reverse phase column chromatography using acetonitrile/water to obtain O-ethyl S-(4-(4-isopropylpiperazin-1-yl)phenyl)carbonodithioate (Chemical Formula **3-3**) (yield 38%).
¹H NMR (CDCl₃, 400 MHz) δ 7.34(d, J=8.8Hz, 2H), 6.94(d, J=8.8Hz, 2H), 4.64(q, 2H), 3.32(m, 4H), 2.70(m, 5H), 1.38(t, 3H), 1.12(d, J=6.4Hz, 6H)

### 3.2. Synthesis of 5-((4-(4-isopropylpiperazin-1-yl)phenyl)thio)-2-nitroaniline (Chemical Formula 4-3)

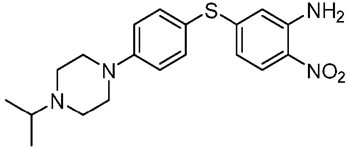

To a solution of O-ethyl S-(4-(4-isopropylpiperazin-1-yl)phenyl)carbonodithioate (Chemical Formula **3-3,** 1.62 g, 5 mmol) in THF/MeOH (30 mL/15 mL) were added 5-chloro-2-nitroaniline (1.72 g, 10 mmol) and CH₃ONa (5M in MeOH, 4.0 mL, 20.28 mmol). The reaction mixture was reacted at 80°C for 6 h. After the reaction was completed, the pH of the resulting mixture was adjusted to pH 7-8 with sodium hydroxide and an aqueous acetic acid solution and the resulting mixture was extracted with methylene chloride and dried. Then, the solvent was removed. The resulting mixture was separated and purified by silica gel column chromatography with Hexane/Ethylacetate 4:2 and DCM/MeOH 10:1 to produce 5-((4-(4-isopropylpiperazin-1-yl)phenyl)thio)-2-nitroaniline (Chemical Formula **4-3**) as a light yellow solid (yield 63%).
¹H NMR (CDCl₃, 400 MHz) δ 7.98(d, J=8.8Hz, 1H), 7.44(d, J=8.8Hz, 2H), 6.96(d, J=8.8Hz, 2H), 6.42(m, 1H), 6.26(s, 1H), 6.02(m, 2H), 3.33(m, 4H), 2.78(m, 1H), 2.71(m, 4H), 1.11(d, J=6.4Hz, 6H)

### 3.3. Synthesis of methyl (5-((4-(4-isopropylpiperazin-1-yl)phenyl)thio)-1H-benzo[d]imidazol-2-yl)carbamate (Chemical Formula 1-3)

### (1) Synthesis of 4-((4-(4-isopropylpiperazin-1-yl)phenyl)thio)benzene-1,2-diamine (Chemical Formula 5-3)

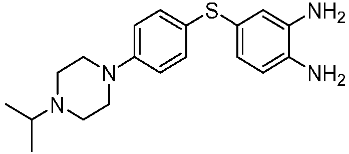

To a solution of 5-((4-(4-isopropylpiperazin-1-yl)phenyl)thio)-2-nitroaniline (Chemical Formula **4-3,** 400 mg, 1.07 mmol) in acetic acid (7 mL) was added activated zinc powder (400 mg), and the mixture was reacted at room temperature for 16 h. The reaction mixture was filtered, the filtrate was evaporated under reduced pressure to remove excess acetic acid, the pH was slightly alkalized with sodium bicarbonate, and then the filtrate was extracted with ethyl acetate and purified to obtain 4-((4-(4-isopropylpiperazin-1-yl)phenyl)thio)benzene-1,2-diamine (Formula **5-3,** 333 mg) as a yellow solid (yield 91%).
¹H NMR (CDCl₃, 400 MHz) δ 7.33(m, 4H), 7.24(d, J=8.4Hz, 2H), 6.84(d, J=8.4Hz, 2H), 6.74(m, 2H), 6.64(m, 1H), 3.40(m, 4H), 3.34(m, 1H), 3.12(m, 4H), 1.28(d, J=6.4Hz, 6H)

### (2) Synthesis of methyl (5-((4-(4-isopropylpiperazin-1-yl)phenyl)thio)-1H-benzo[d]imidazol-2-yl)carbamate (Chemical Formula 1-3)

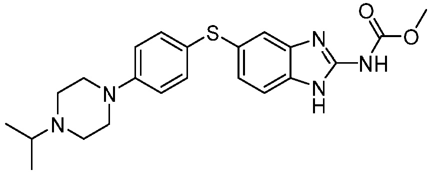

To a solution of 4-((4-(4-isopropylpiperazin-1-yl)phenyl)thio)benzene-1,2-diamine (Chemical Formula **5-3,** 150 mg, 0.44 mmol) in 5% acetic acid ethyl alcohol (3 mL), was added 1,3-bis(methoxycarbonyl)-S-methylisothiourea (300 mg, 1.45 mmol) and the reaction mixture was heated and refluxed for 24 h. The reaction mixture was extracted with ethyl acetate, separated and purified by silica gel column chromatography using a mixed solvent of ethyl acetate and hexane to obtain methyl (5-((4-(4-isopropylpiperazin-1-yl)phenyl)thio)-1H-benzo[d]imidazol-2-yl)carbamate (Chemical Formula **1-3,** 110 mg) as a product (yield 59%).
¹H-NMR (DMSO-d6, 400 MHz) δ 11.74(m, 2H), 7.34(m, 1H), 7.28(s, 1H), 7.22(d, J= 8.8 Hz, 2H), 7.03(m, 1H), 6.92(d, J = 8.8 Hz, 2H), 3.74(s, 3H), 3.12(m, 4H), 2.66(m, 1H), 2.56(m, 4H), 1.00(d, J = 6.4 Hz, 6H)

### Example 4. Synthesis of Chemical Formula 1-4

The synthesis process of the thiobenzimidazole derivative of Chemical Formula 1-4 is shown by Reaction Scheme 4 presented below.

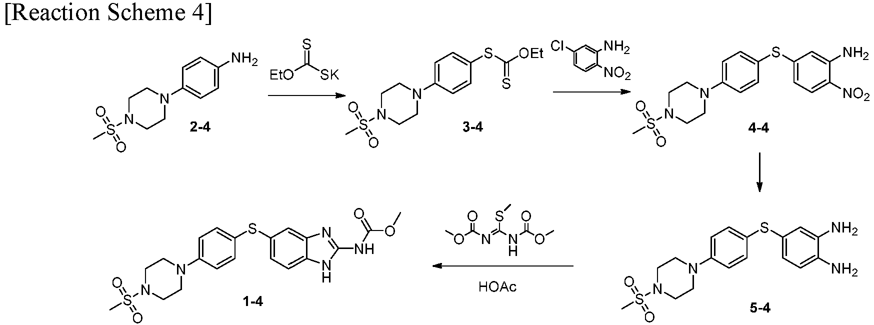

### 4.1. Synthesis of O-ethyl S-(4-(4-(methylsulfonyl)piperazin-1-yl)phenyl)carbonodithioate (Chemical Formula 3-4)

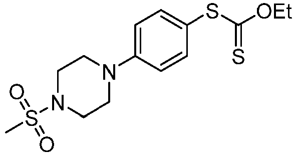

A mixed solution of 4-(4-(methylsulfonyl)piperazin-1-yl)aniline (Chemical Formula **2-4,** 1.5 g, 5.87 mmol) in THF/MeOH (17/30 mL), sodium nitrite (0.82 g, 11.74 mmol) dissolved in distilled water (6 mL), and concentrated hydrochloric acid (1.0 mL) was prepared at 0°C in an ice bath. To the mixture prepared previously was slowly added dropwise a solution of potassium ethylxanthate (6.0 g, 37.4 mmol) in distilled water (15 mL) at 40°C, then the mixture was reacted for 1 h. After the reaction was completed, the mixture was poured into water (150 mL) and extracted with EA (150 mL×2). The organic layer was dried over anhydrous magnesium sulfate, concentrated, and purified with ethyl acetate and n-hexane to obtain O-ethyl S-(4-(4-(methylsulfonyl)piperazin-1-yl)phenyl)carbonodithioate (Chemical Formula **3-4,** 1.18 g) as a brown solid (yield 56%).
¹H NMR (CDCl₃, 400 MHz) δ 7.42(d, J=8.8Hz, 1H), 7.32(m, 1H), 6.97(m, 2H), 4.65(q, 2H), 3.38(m, 6H), 3.32(m, 2H), 2.86(s, 3H), 1.37(t, 3H)

### 4.2. Synthesis of 5-((4-(4-(methylsulfonyl)piperazin-1-yl)phenyl)thio)-2-nitroaniline (Chemical Formula 4-4)

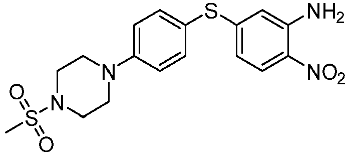

To a solution of O-ethyl S-(4-(4-(methylsulfonyl)piperazin-1-yl)phenyl)carbonodithioate (Chemical Formula **3-4,** 0.6 g, 1.66 mmol) in THF/MeOH (15/20 mL) was added 5-chloro-2-nitroaniline (0.314 g, 1.82 mmol), then sodium hydroxide powder (0.279 g, 6.97 mmol) dissolved in distilled water (5 mL) was added. The reaction mixture was reacted at 90°C for 4 h, then separated and purified by a mixed solution of ethyl acetate and hexane to obtain 5-((4-(4-(methylsulfonyl)piperazin-1-yl)phenyl)thio)-2-nitroaniline (Chemical Formula **4-4,** 0.494 g) as a yellow solid (yield 51%).
¹H NMR (CDCl₃, 400 MHz) δ 7.99(d, J=9.2Hz, 1H), 7.48(d, J=8.8Hz, 2H), 7.00(d, J=8.8Hz, 2H), 6.42(m, 1H), 6.30(s, 1H), 6.03(s, 2H), 3.42(s, 8H), 2.87(s, 3H),

### 4.3. Synthesis of methyl (5-((4-(4-(methylsulfonyl)piperazin-1-yl)phenyl)thio)-1H-benzo[d]imidazol-2-yl)carbamate (Chemical Formula 1-4)

### (1) Synthesis of 4-((4-(4-(methylsulfonyl)piperazin-1-yl)phenyl)thio)benzene-1,2-diamine (Chemical Formula 5-4)

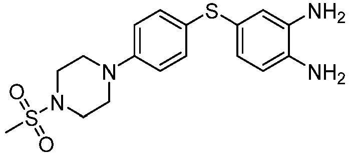

To a solution of 5-((4-(4-(methylsulfonyl)piperazin-1-yl)phenyl)thio)-2-nitroaniline (Chemical Formula **4-4,** 0.330 g, 0.81 mmol) in acetic acid (4 mL) was added activated zinc powder (250 mg), and the mixture was reacted at room temperature for 10 h. The reaction mixture was filtered, the filtrate was evaporated under reduced pressure to remove excess acetic acid, the pH was slightly alkalized with sodium bicarbonate solution, and then the filtrate was extracted with ethyl acetate and purified to obtain 4-((4-(4-(methylsulfonyl)piperazin-1-yl)phenyl)thio)benzene-1,2-diamine (Chemical Formula **5-4**, 210 mg) as a yellow solid (yield 69%).
¹H NMR (CDCl₃, 400 MHz) δ 7.25(d, J=8.8Hz, 2H), 6.86(d, J=8.8Hz, 2H), 6.77(m, 2H), 6.67(d, J=8.0Hz, 1H), 3.78(m, 8H), 3.40(m, 4H), 2.84(s, 3H),

### (2) Synthesis of methyl (5-((4-(4-(methylsulfonyl)piperazin-1-yl)phenyl)thio)-1H-benzo[d]imidazol-2-yl)carbamate (Chemical Formula 1-4)

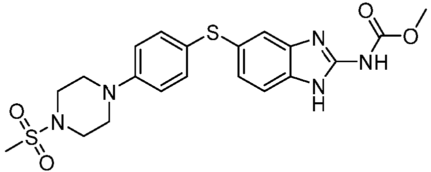

To a solution of 4-((4-(4-(methylsulfonyl)piperazin-1-yl)phenyl)thio)benzene-1,2-diamine (Chemical Formula **5-4,** 140 mg, 0.37 mmol) in 5% acetic acid ethyl alcohol (5 mL), was added 1,3-bis(methoxycarbonyl)-S-methylisothiourea (300 mg, 1.45 mmol) and the mixture was reacted at 110°C for 5 h. At this time, the insoluble reactants were dissolved. As the reaction time elapsed, a white precipitate was obtained, and methyl alcohol (50 mL) was added at 50°C and the precipitate was filtered to obtain the product. Then, the product was washed with methyl alcohol (100 mL) until the urea odor was removed, to obtain methyl (5-((4-(4-(methylsulfonyl)piperazin-1-yl)phenyl)thio)-1H-benzo[d]imidazol-2-yl)carbamate (Chemical Formula **1-4,** 170 mg) as a pure product (100% yield).
¹H NMR (DMSO-d₆, 400 MHz) δ 11.89(m, 1H), 11.32(m, 1H), 7.36(m, 2H), 7.23(d, J=8.8Hz, 2H), 7.06(m, 1H), 6.98(d, J=8.8Hz, 2H), 3.74(s, 3H), 3.25(m, 8H), 2.92(s, 3H)

### Example 5. Synthesis of Chemical Formula 1-5

The synthesis process of the thiobenzimidazole derivative of Chemical Formula 1-5 is shown by Reaction Scheme 5 presented below.

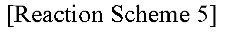

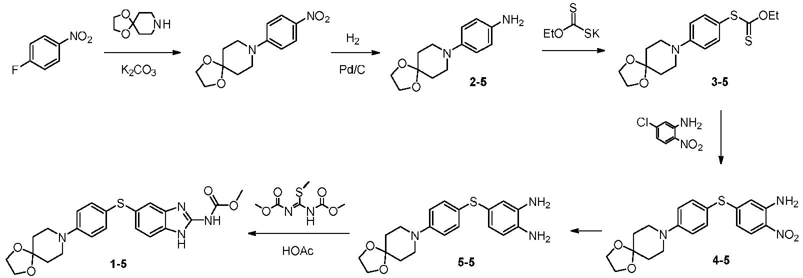

### 5.1. Synthesis of S-(4-(1,4-dioxa-8-azaspiro[4.5]decan-8-yl)phenyl) O-ethyl carbonodithioate (Chemical Formula 3-5)

### (1) Synthesis of 4-(1,4-dioxa-8-azaspiro[4.5]decan-8-yl)aniline (Chemical Formula 2-5)

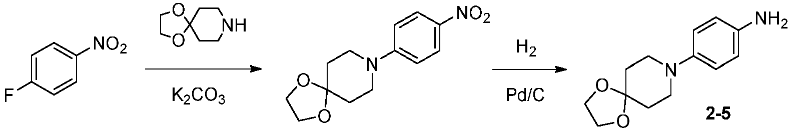

To a solution of 1-fluoro-4-nitrobenzene (1.41 g, 0.01 mol) in DMF (10 mL), were added 1,4-dioxa-8-azaspiro[4.5]decane (2.14 g, 0.015 mol) and potassium carbonate (2.76 g, 0.02 mol), and the mixture was reacted at 100°C for 14 h. The solid was removed from the reaction mixture at high temperature, then cooled to room temperature. The resulting solid precipitate was filtered to obtain 8-(4-nitrophenyl)-1,4-dioxa-8-azaspiro[4.5]decane (2.34 g) as a light yellow solid. To the methanol (70 mL) solution of the resulting solid was added 10% of Pd/C (500 mg). Hydrogen gas was injected under pressure. Reaction was performed in a Par reactor for 18 h. The reaction product was filtered and the filtrate was concentrated to obtain 4-(1,4-dioxa-8-azaspiro[4.5]decan-8-yl)aniline (Chemical Formula **2-5,** 1.522g) as a light yellow solid (yield 65%).

### (2) Synthesis of S-(4-(1,4-dioxa-8-azaspiro[4.5]decan-8-yl)phenyl) O-ethyl carbonodithioate (Chemical Formula 3-5)

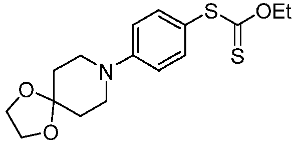

A mixed solution of 4-(1,4-dioxa-8-azaspiro[4.5]decan-8-yl)aniline (Chemical Formula **2-5,** 1.1 g, 4.69 mmol) in distilled water (15 mL), sodium nitrite (470 mg) dissolved in distilled water (5 mL), and concentrated hydrochloric acid (0.8 mL) was prepared at 0°C in an ice bath. To the mixture prepared previously, a solution of Potassium ethylxanthate (1.24 g, 7.48 mmol) in distilled water (3 mL) was slowly added dropwise at 50°C and the mixture was reacted for 1 h.

After the reaction was completed, the mixture was poured into water (70 mL) and extracted with EA (70 mL×2). The organic layer was dried over anhydrous magnesium sulfate, concentrated, and purified with ethyl acetate and n-hexane to obtain S-(4-(1,4-dioxa-8-azaspiro[4.5]decan-8-yl)phenyl) O-ethyl carbonodithioate (Chemical Formula **3-5,** 1.11 g) as a brown solid (yield 68%).
¹H NMR (CDCl₃, 400 MHz) δ 7.36(d, J=8.8Hz, 2H), 6.95(d, J=8.8Hz, 2H), 4.64(q, 2H), 3.45(m, 4H), 1.84(m, 4H),1.36(t, 3H)

### 5.2. Synthesis of 5-((4-(1-oxa-8-azaspiro[4.5]decan-8-yl)phenyl)thio)-2-nitroaniline (Chemical Formula 4-5)

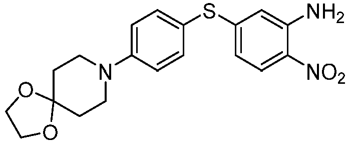

To a solution of S-(4-(1,4-dioxa-8-azaspiro[4.5]decan-8-yl)phenyl) O-ethyl carbonodithioate (Chemical Formula **3-5,** 770 mg, 2.27 mmol) in THF/MeOH (23/38 mL) was added 5-chloro-2-nitroaniline (400 mg, 3.31 mmol), then sodium hydroxide powder (340 mg) dissolved in distilled water (7.6 mL) was added. The reaction mixture was reacted at 90°C for 5 h, then separated and purified by a mixed solution of ethyl acetate and hexane to obtain 5-((4-(1-oxa-8-azaspiro[4.5]decan-8-yl)phenyl)thio)-2-nitroaniline (Chemical Formula **4-5,** 470 mg) as a yellow solid (yield 54%).
¹H NMR (CDCl₃, 400 MHz) δ 7.98(d, J=9.2Hz, 1H), 7.42(d, J=8.8Hz, 2H), 6.97(d, J=8.8Hz, 2H), 6.42(m, 1H), 6.25(s, 1H), 6.03(s, 2H), 4.15(s, 4H), 3.47(m, 4H), 1.86(m, 4H),

### 5.3. Synthesis of methyl (5-((4-(1,4-dioxa-8-azaspiro[4.5]decan-8-yl)phenyl)thio)-1H-benzo[d]imidazol-2-yl)carbamate (Chemical Formula 1-5)

### (1) Synthesis of 4-((4-(1,4-dioxa-8-azaspiro[4.5]decan-8-yl)phenyl)thio)benzene-1,2-diamine (Chemical Formula 5-5)

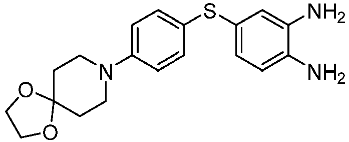

To a solution of 5-((4-(1-oxa-8-azaspiro[4.5]decan-8-yl)phenyl)thio)-2-nitroaniline (Chemical Formula **4-5**, 450 mg, 1.17 mmol) in acetic acid (7 mL) was added activated zinc powder (450 mg), and the mixture was reacted at room temperature for 7 h. The reaction mixture was filtered, the filtrate was evaporated under reduced pressure to remove excess acetic acid, the pH was slightly alkalized with saturated sodium bicarbonate solution, and then the filtrate was extracted and purified with ethyl acetate to obtain 4-((4-(1,4-dioxa-8-azaspiro[4.5]decan-8-yl)phenyl)thio)benzene-1,2-diamine (Chemical Formula **5-5,** 403 mg) as a yellow solid (yield 97%).
¹HNMR (CDCl₃, 400 MHz) δ 7.33(d, J=8.8Hz, 2H), 7.08(s, 1H), 6.93(m, 1H), 6.88(d, J=8.8Hz, 2H), 6.63(d, J=9.4Hz, 1H), 4.01(s, 4H), 3.35(m, 4H), 1.85(m, 4H)

### (2) Synthesis of methyl (5-((4-(1,4-dioxa-8-azaspiro[4.5]decan-8-yl)phenyl)thio)-1H-benzo[d]imidazol-2-yl)carbamate (Chemical Formula 1-5)

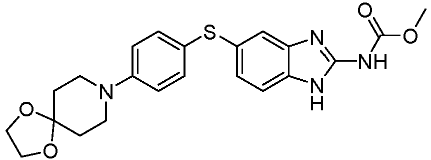

To a solution of 4-((4-(1,4-dioxa-8-azaspiro[4.5]decan-8-yl)phenyl)thio)benzene-1,2-diamine (Chemical Formula **5-5**, 350 mg, 0.98 mmol) in 5% acetic acid ethyl alcohol (7 mL) was added 1,3-bis(methoxycarbonyl)-S-methylisothiourea (509 mg, 2.47 mmol) and the mixture was reacted at 80°C for 24 h. As the reaction time elapsed, a white precipitate was obtained from the reactants. Methyl alcohol (20 mL) was added at 40°C and the precipitate was filtered to obtain the product. Then, the product was washed with methyl alcohol until the urea odor was removed, to obtain (5-((4-(1,4-dioxa-8-azaspiro[4.5]decan-8-yl)phenyl)thio)-1H-benzo[d]imidazol-2-yl)carbamate (Chemical Formula **1-5,** 210 mg) as a pure product (yield 49%).
¹H NMR (DMSO-d₆, 400 MHz) δ 11.80(m, 1H), 11.32(m, 1H), 7.33(m, 2H), 7.18(d, J = 8.4 Hz, 2H), 7.04(m, 1H), 6.93(d, J = 8.8 Hz, 2H), 3.90(s, 4H), 3.74(s, 3H), 3.28(m, 4H), 1.68(m, 4H)

### Example 6. Synthesis of Chemical Formula 1-6

The synthesis process of the thiobenzimidazole derivative of Chemical Formula 1-6 is shown by Reaction Scheme 6 presented below.

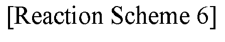

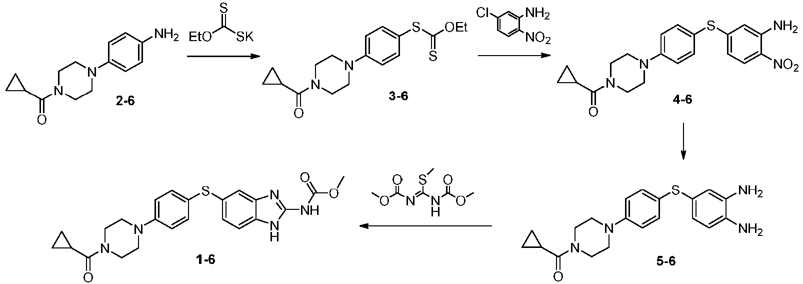

### 6.1. Synthesis of S-(4-(4-(cyclopropanecarbonyl)piperazin-1-yl)phenyl) O-ethyl carbonodithioate (Chemical Formula 3-6)

A mixed solution of (4-(4-aminophenyl)piperazin-1-yl)(cyclopropyl)methanone (Chemical Formula **2-6,** 1.22 g, 5.0 mmol) in distilled water (15 mL), sodium nitrite (525 mg) dissolved in distilled water (5 mL), and concentrated hydrochloric acid (0.995 mL) was prepared at 0°C in an ice bath. To the mixture prepared previously, a solution of Potassium ethylxanthate (1.552 g, 9.5 mmol) in distilled water (3 mL) was slowly added dropwise at 50°C and the mixture was reacted for 1 h.

After the reaction was completed, the mixture was poured into water (800 mL) and extracted with EA (80 mL×2). The organic layer was dried over anhydrous magnesium sulfate, concentrated, and purified with ethyl acetate and n-hexane to obtain S-(4-(4-(cyclopropanecarbonyl)piperazin-1-yl)phenyl) O-ethyl carbonodithioate (Chemical Formula 3-6, 450 mg) as a brown solid (yield 26%).
¹H NMR (CDCl₃, 400 MHz) δ 7.40(d, J=8.8Hz, 2H), 6.93(d, J=8.8Hz, 2H), 4.64(q, 2H), 3.87(m, 4H), 3.37(m, 4H), 1.77(m, 1H), 1.37(t, 3H), 1.06(m, 2H), 0.84(m, 2H)

### 6.2. Synthesis of (4-(4-((3-amino-4-nitrophenyl)thio)phenyl)piperazin-1-yl)(cyclopropyl)methanone (Chemical Formula 4-6)

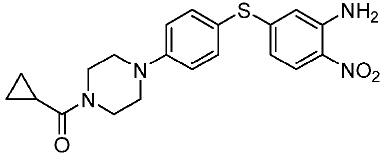

To a solution of S-(4-(4-(cyclopropanecarbonyl)piperazin-1-yl)phenyl)O-ethyl carbonodithioate (Chemical Formula **3-6,** 300 mg, 0.86 mmol) in THF/MeOH (10/15 mL) was added 5-chloro-2-nitroaniline (177 mg, 1.03 mmol), then sodium hydroxide powder (170 mg) dissolved in distilled water (4 mL) was added. The reaction mixture was reacted at 90°C for 5 h, then separated and purified by a mixed solution of ethyl acetate and hexane (3:1, v/v) to obtain (4-(4-((3)-amino-4-nitrophenyl)thio)phenyl)piperazin-1-yl)(cyclopropyl)methanone (Chemical Formula **4-6,** 150 mg) as a yellow solid (yield 44%).
¹H NMR (CDCl₃, 400 MHz) δ 7.98(d, J=9.2Hz, 1H), 7.45(d, J=8.8Hz, 2H), 6.69(d, J=8.8Hz, 2H), 6.40(m, 1H), 6.29(s, 2H), 6.04(s, 2H), 3.89(m, 4H), 3.37(m, 4H), 1.78(m, 1H), 1.05(m, 2H), 0.84(m, 2H)

### 6.3. Synthesis of methyl (5-((4-(4-acetylpiperidin-1-yl)phenyl)thio)-1H-benzo[d]imidazol-2-yl)carbamate (Chemical Formula 1-6)

### (1) Synthesis of cyclopropyl(4-(4-((3,4-diaminophenyl)thio)phenyl)piperazin-1-yl)methanone (Chemical Formula 5-6)

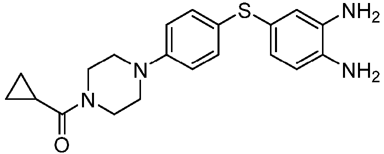

To a solution of (4-(4-((3-amino-4-nitrophenyl)thio)phenyl)piperazin-1-yl)(cyclopropyl)methanone (Chemical Formula **4-6,** 150 mg, 0.37 mmol) in acetic acid (3 mL), was added activated zinc powder (250 mg) and the mixture was reacted at room temperature for 12 h. The reaction mixture was filtered, the filtrate was evaporated under reduced pressure to remove an excess of acetic acid, the pH was slightly alkalized with saturated sodium bicarbonate solution, and then the filtrate was extracted and purified with ethyl acetate to obtain cyclopropyl(4-(4-((3,4)-diaminophenyl)thio)phenyl)piperazin-1-yl)methanone (Chemical Formula **5-6,** 120 mg) as a yellow solid (yield 87%).
¹H NMR (CDCl₃, 400 MHz) δ 7.23(s, 1H), 7.04(d, J=8.8Hz, 2H), 6.58(d, J=8.8Hz, 2H), 6.43(d, J=9.4Hz, 2H), 4.94(br. s, 4H), 3.56(m, 4H), 3.35(m, 4H), 1.41(m, 1H), 0.78 (m, 2H), 0.53(m, 2H)

### (2) Synthesis of methyl (5-((4-(4-acetylpiperidin-1-yl)phenyl)thio)-1H-benzo[d]imidazol-2-yl)carbamate (Chemical Formula 1-6)

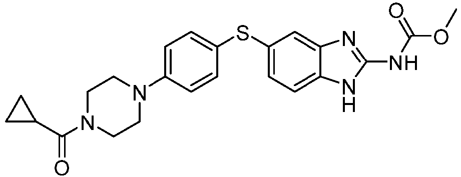

To a solution of cyclopropyl(4-(4-((3,4-diaminophenyl)thio)phenyl)piperazin-1-yl)methanone (Chemical Formula **5-6,** 70 mg, 0.19 mmol) in 5% acetic acid ethyl alcohol (1.5 mL) was added 1,3-bis(methoxycarbonyl)-S-methylisothiourea (110 mg, 0.53 mmol), then the mixture was heated to reflux and reacted for 5 h. Methyl alcohol (7 mL) was added to the reactants at 40°C, then the reaction mixture was filtered, and washed with additional methyl alcohol until the urea odor was removed, to obtain methyl (5-((4-(4-acetylpiperidin-1-yl))phenyl)thio)-1H-benzo[d]imidazol-2-yl)carbamate (Chemical Formula **1-6,** 70 mg) as a pure product (yield 81.6%).
¹H NMR (DMSO-d₆, 400 MHz) δ 11.86(m, 1H), 11.43(m, 1H), 7.33(m, 2H), 7.23(d, J=8.8Hz, 2H), 7.05(m, 1H), 6.94(d, J=8.8Hz, 2H), 3.80(s, 2H), 3.74(s, 3H), 3.59(s, 2H), 3.17(m, 4H), 2.02(m, 1H), 0.75(m, 4H)

### Example 7. Synthesis of Chemical Formula 1-7

The synthesis process of the thiobenzimidazole derivative of Chemical Formula 1-7 is shown by Reaction Scheme 7 presented below.

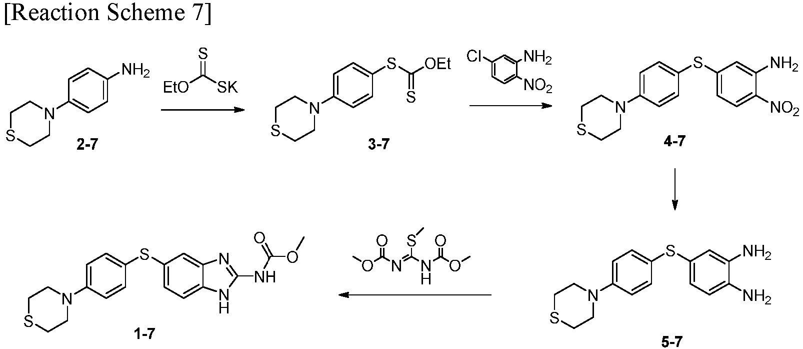

### 7.1. Synthesis of O-ethyl S-(4-thiomorpholinophenyl)carbonodithioate (Chemical Formula 3-7)

A mixed solution of 4-(4-Thiomorpholinyl)benzenamine (Chemical Formula 2-7, 1.56 g, 8.0 mmol) in distilled water (20 mL), sodium nitrite (672 mg) dissolved in distilled water (5 mL), and concentrated hydrochloric acid (1.2 mL) was prepared at 0°C in an ice bath. To the mixture prepared previously, a solution of Potassium ethylxanthate (1.94 g, 12.1 mmol) in distilled water (3 mL) was slowly added dropwise at 50°C and the mixture was reacted for 1 h.

After the reaction was completed, the mixture was poured into water (100 mL) and extracted with EA (100 mL×2). The organic layer was dried over anhydrous magnesium sulfate, concentrated, and purified with ethyl acetate and n-hexane to obtain O-ethyl S-(4-thiomorpholinophenyl)carbonodithioate (Chemical Formula 3-7, 600 mg) as a brown solid (yield 75%).
¹H NMR (CDCl₃, 400 MHz) δ 7.38(d, J=8.4Hz, 2H), 6.90(d, J=8.8Hz, 2H), 4.65(q, 2H), 3.71(m, 4H), 2.76(m, 4H), 1.37(t, 3H)

### 7.2. Synthesis of 2-nitro-5-((4-thiomorpholinophenyl)thio)aniline (Chemical Formula 4-7)

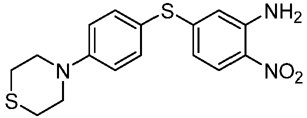

To a solution of O-ethyl S-(4-thiomorpholinophenyl)carbonodithioate (Chemical Formula **3-7,** 600 mg, 2.0 mmol) in THF/MeOH (20/30 mL) was added 5-chloro-2-nitroaniline (346 mg, 2.1 mmol), then sodium hydroxide powder (336 mg) dissolved in distilled water (7 mL) was added. The reaction mixture was reacted at 90°C for 5 h, then separated and purified by a mixed solution of ethyl acetate and hexane to obtain 2-nitro-5-((4-thiomorpholinophenyl)thio)aniline (Chemical Formula **4-7,** 500 mg) as a yellow solid (yield 72%).
¹H NMR (CDCl₃, 400 MHz) δ 7.99(d, J=8.8Hz, 1H), 7.42(d, J=8.8Hz, 2H), 6.93(d, J=8.4Hz, 2H), 6.42(m, 1H), 6.28(s, 1H), 6.03(s, 2H), 3.72(m, 4H), 2.77(m, 4H)

### 7.3. Synthesis of methyl (5-((4-thiomorpholinophenyl)thio)-1H-benzo[d]imidazol-2-yl)carbamate (Chemical Formula 1-7)

### (1) Synthesis of 4-((4-thiomorpholinophenyl)thio)benzene-1,2-diamine (Chemical Formula 5-7)

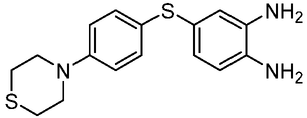

To a solution of 2-nitro-5-((4-thiomorpholinophenyl)thio)aniline (Chemical Formula **4-7**, 500 mg, 1.44 mmol) in acetic acid (8 mL) was added activated zinc powder (500 mg) and the mixture was reacted at room temperature for 5 h. The reaction mixture was filtered, the filtrate was evaporated under reduced pressure to remove an excess of acetic acid, the pH was slightly alkalized with sodium bicarbonate, and then the filtrate was extracted and purified with ethyl acetate to obtain 4-((4-thiomorpholinophenyl)thio)benzene-1,2-diamine (Chemical Formula 5-7, 398 mg) as a yellow solid (yield 87%).
¹H NMR (CDCl₃, 400 MHz) δ 7.83(d, J=8.8Hz, 2H), 7.40(d, J=8.8Hz, 2H), 7.20 and 6.93(2d, J=8.4Hz, 2H), 6.28(s, 1H), 4.95(br, s, 4H), 3.72(m, 4H), 2.67(m, 4H)

### (2) Synthesis of methyl (5-((4-thiomorpholinophenyl)thio)-1H-benzo[d]imidazol-2-yl)carbamate (Chemical Formula 1-7)

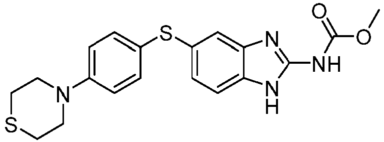

To a solution of 4-((4-thiomorpholinophenyl)thio)benzene-1,2-diamine (Chemical Formula **5-7,** 158 mg, 0.5 mmol) in 5% acetic acid ethyl alcohol (3 mL) was added 1,3-bis(methoxycarbonyl)-S-methylisothiourea (260 mg, 1.26 mmol) and the mixture was reacted at 80°C for 24 h. As the reaction time elapsed, a white precipitate was obtained from the reactants. Methyl alcohol (20 mL) was added at 40°C and the mixture was filtered to obtain a product. The product was washed with methyl alcohol until the urea odor was removed to obtain methyl (5-((4-thiomorpholinophenyl)thio)-1H-benzo[d]imidazol-2-yl)carbamate (Chemical Formula **1-7,** 120 mg) as a pure product (yield 60.3%).
¹H NMR (DMSO-d6, 400 MHz) δ 11.83(m, 1H), 11.38(m, 1H), 7.33(m, 2H), 7.22(d, J=8.4Hz, 2H), 7.04(d, J=8.0Hz, 1H), 6.92(d, J=Hz, 2H), 3.74(s, 3H), 3.56(m, 4H), 2.63(m, 4H)

### Example 8. Synthesis of Chemical Formula 6-1

The synthesis process of the thiobenzimidazole derivative of Chemical Formula 6-1 is shown by Reaction Scheme 8 presented below.

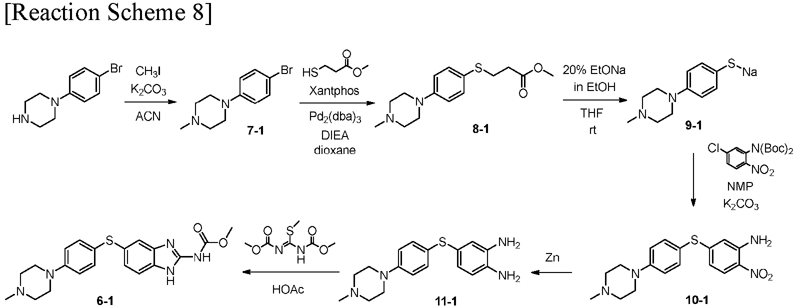

### 8.1. Synthesis of 1-(4-bromophenyl)-4-methylpiperazine (Chemical Formula 7-1)

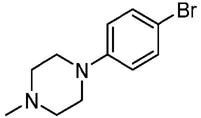

To a solution of 1-(4-bromophenyl)piperazine (5.0 g, 20.73 mmol, 1.0 eq) in anhydrous acetonitrile (25 mL), was added methyl iodide (3.53 mL, 24.88 mmol, 1.2 eq) and K₂CO₃ (5.73 g, 41.46 mmol, 2.0 eq). After the addition was completed, the reaction mixture was stirred at 70°C under an N₂ atmosphere for 3 h. The reaction mixture was filtered and the filtrate was concentrated. The residue was purified by silica gel (HE:EtOAC=5:1, v/v) column chromatography to obtain the compound 1-(4-bromophenyl)-4-methylpiperazine (Chemical Formula **7-1**, 4.34 g, yield 82%) as a white solid.

¹H NMR (400 MHz, CDCl₃) δ 7.23 (d, J=8.4 Hz, 2H), 6.64 (d, J=8.8 Hz, 2H), 3.54 (m, 4H), 2.36 (m, 4H), 2.25 (s, 3H).

### 8.2. Synthesis of methyl 3-((4-(4-methylpiperazin-1-yl)phenyl)thio)propanoate (Chemical Formula 8-1)

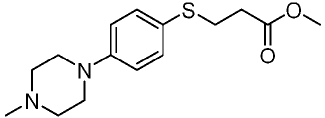

To a solution of 1-(4-bromophenyl)-4-methylpiperazine (Formula **7-1,** 4.45 g, 17.45 mmol, 1.0 eq) in dry dioxane (25 mL), was added methyl 3-mercaptopropanoate (13.6 mL, 122.2 mmol, 7.0 eq), xantphos (2.02 g, 3.49 mmol, 0.2 eq). After the addition of DIEA (9.15 mL, 52.35 mmol, 3.0 eq) and Pd₂(dba)₃ (1.6 g, 1.75 mmol, 0.1 eq) was completed, the reaction mixture was stirred at 110°C under an N₂ atmosphere for 17 h. The reaction mixture was filtered and the filtrate was concentrated. The residue was dissolved in a small amount of methylene chloride and ethyl acetate, and purified by silica gel column chromatography (Hexane: EtOAC = 2:1, v/v) at an appropriate concentration to obtain the compound methyl 3-((4-(4-methylpiperazin-1-yl)phenyl)thio)propanoate (Chemical Formula **8-1, 4.57** g, yield 89%) as a yellow oil.

¹H NMR (400 MHz, CDCl₃) δ 7.38 (d, J=8.6 Hz, 2H), 6.95 (d, J=8.6 Hz, 2H), 3.61 (s, 3H), 3.44 (m, 4H), 3.15 (t, J =4.2 Hz, 2H), 2.64 (s, J = 4.2 Hz, 2H), 2.36 (m, 4H), 2.25 (s, 3H).

### 8.3. Synthesis of sodium 4-(4-methylpiperazin-1-yl)benzenethiolate (Chemical Formula 9-1)

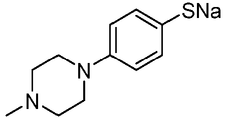

To a solution of 3-((4-(4-methylpiperazin-1-yl)phenyl)thio)propanoate (Chemical Formula **8-1**, 2.68 g, 9.1 mmol, 1.0 eq.) in dry THF (50 mL), was added 20% Sodium ethoxide (4.64 g, 13.65 mmol, 1.5 eq.) ethanol, and then the reaction mixture was allowed to react at room temperature for 30 m. After the reaction was completed, the mixture was concentrated to obtain the compound sodium 4-(4-methylpiperazin-1-yl)benzenethiolate (Chemical Formula 9-1, 1.8 g, yield 86%) as a brown solid.

¹H NMR (400 MHz, CDCl₃) δ 7.20 (d, J=8.4 Hz, 2H), 6.55 (d, J=8.6 Hz, 2H), 3.37 (m, 4H), 2.40 (m, 4H), 2.21 (s, 3H).

### 8.4. Synthesis of 5-((4-(4-methylpiperazin-1-yl)phenyl)thio)-2-nitroaniline (Chemical Formula 10-1)

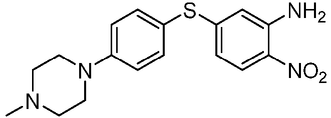

Sodium 4-(4-methylpiperazin-1-yl)benzenethiolate (Chemical Formula 9-1, 1.8 g, 7.81 mmol, 1.0 eq.) was dissolved in dry N-methylpyrrolidinedimethyl (40 mL). To the solution was sequentially added tert-butyl (tert-butoxycarbonyl)(5-chloro-2-nitrophenyl)carbamate (2.91 g, 7.81 mmol, 1.0 eq.) and K₂CO₃ (3.23 g, 23.43 mmol, 3.0 eq.), and the reaction mixture was stirred at 130°C for 18 h under an N₂ atmosphere.

After the reaction was completed, the residue was treated with diluted hydrochloric acid and purified by silica gel column chromatography (DCM: MeOH = 20:1, v/v) to obtain 5-((4-(4-methylpiperazin-1-yl)phenyl)thio)-2-nitroaniline (Chemical Formula 10-1, 2.30 mg, yield 86%) as an orange solid.
¹H NMR (400 MHz, DMSO-d₆) δ 7.42 (br. s, 2H), 7.82 - 6.53 (m, 7H, 2X aromatic H), 3.64 (m, 4H), 2.40 (m, 4H), 2.21 (s, 3H)

### 8.5. Synthesis of 4-((4-(4-methylpiperazin-1-yl)phenyl)thio)benzene-1,2-diamine (Chemical Formula 11-1)

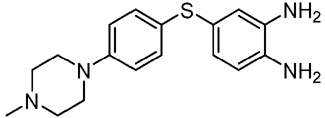

To a solution of 5-((4-(4-methylpiperazin-1-yl)phenyl)thio)-2-nitroaniline (Chemical Formula 10-1, 2.3 g, 6.68 mmol, 1.0 eq.) in acetic acid (330 mL) was added activated zinc powder (1.9 g), and the mixture was reacted at room temperature for 10 h. The reaction mixture was filtered, the filtrate was evaporated under reduced pressure to remove excess acetic acid, the pH was slightly alkalized with saturated sodium bicarbonate solution, and then the filtrate was extracted with ethyl acetate and purified to obtain 4-((4-(4-methylpiperazin-1-yl)phenyl)thio)benzene-1,2-diamine (Chemical Formula 11-1, 1.0 g, yield 48%) as a yellow solid.
¹H NMR (400 MHz, DMSO-d6): δ 7.23 (s, 1H), 7.03 (d, J=8.8 Hz, 2H), 6.84 (d, J=8.8 Hz, 2H), 7.20 (d, J = 7.2 Hz, 1H), 6.45 (d, J = 7.2 Hz, 1H), 3.44 (m, 4H), 2.37 (m, 4H), 2.20 (s, 3H)

### 8.6. Synthesis of methyl (5-((4-(4-methylpiperazin-1-yl)phenyl)thio)-1H-benzo[d]imidazol-2-yl)carbamate (Chemical Formula 6-1)

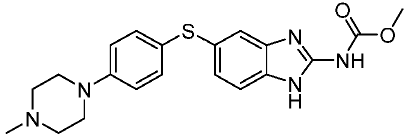

To a solution of 4-((4-(4-methylpiperazin-1-yl)phenyl)thio)benzene-1,2-diamine (Chemical Formula **11-1,** 800 mg, 2.54 mmol, 1.0 eq.) in acetic acid (20 mL), was added 1,3-bis(methoxycarbonyl)-S-methylisothiourea (575 mg, 2.79 mmol, 1.1 eq.) and the mixture was stirred at 80°C for 2 h. It was confirmed by LCMS that the reaction was completed. The resulting reaction mixture was dried over anhydrous Na₂SO₄ and filtered. The filtrate was concentrated, and an aliquot was purified by Prep-TLC to obtain methyl (5-((4-(4-methylpiperazin-1-yl)phenyl)thio)-1H-benzo[d]imidazol-2-yl)carbamate (Chemical Formula 6-1, 632 mg, yield 63%) as a white solid.
¹H NMR (400 MHz, DMSO-d₆) δ 11.90 & 11.70 (2 x br. s, 2H), 7.28-7.32 (m, 2H), 7.20 (d, J=8.8 Hz,2H), 6.99-7.01 (m, 1H), 6.91 (d, J=8.8 Hz, 2H), 3.71 (s, 3H), 3.11-3.14 (m, 4H), 2.41-2.43 (m, 4H), 2.20 (s, 3H)

### 8.7. Synthesis of methyl(5-((4-(4-methylpiperazin-1-yl)phenyl)thio)-1H-benzo[d]imidazol-2-yl)carbamate hydrochloric acid salt

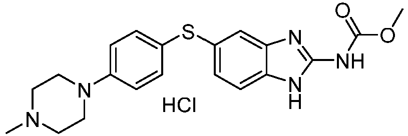

### 8.7.1. Method 1

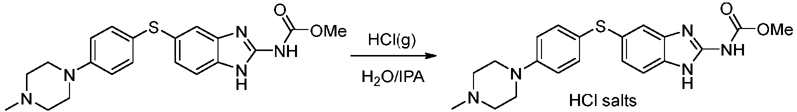

To a solution of methyl (5-((4-(4-methylpiperazin-1-yl)phenyl)thio)-1H-benzo[d]imidazol-2-yl)carbamate (Chemical Formula **6-1,** 370 mg, 0.93 mmol) in methanol (75 mL), hydrogen chloride (HCl) gas was injected at 2-minute intervals until the pH reached 1 or less. The reactant was evaporated under reduced pressure to obtain an oil-form product, then isopropyl alcohol (37 mL) was added. The mixture was dissolved by heating, and then the mixture was cooled to room temperature to produce a solid suspension. Isopropyl ether (35 mL) was added to the solid suspension in portions. The mixture was stirred for 3 h, and then filtered. At this time, the filtrate was washed with a mixed solvent of IPA and IPE, dried with hot air at 50°C for 1 h, and vacuum dried at 40°C for 2 h to obtain 0.350 g of methyl (5-((4-(4-methylpiperazin-1-yl)phenyl)thio)-1H-benzo[d]imidazol-2-yl)carbamate hydrochloric acid salt as an off-white solid (yield 87%).

¹H NMR (D₂O, 400 MHz) δ 2.93(s, 3H), 3.08-3.14 & 3.19-3.25 (2m,4H), 3.61(d, J = 12.5 Hz, 2H), 3.64(d, J = 12.5 Hz, 2H), 3.89(s, 3H), 7.04-7.44(m, 7H).

### 8.7.2. Method 2

A mixed solution of methyl (5-((4-(4-methylpiperazin-1-yl)phenyl)thio)-1H-benzo[d]imidazol-2-yl)carbamate (Chemical Formula **6-1,** 500 mg, 1.257 mmol) in methylene chloride (CH₂Cl₂, 30 mL) and methanol (30 mL) was cooled in an ice bath. To the solution was added 4 moles of hydrochloric acid solution (HCl, 5.03 mmol, 4 eq.) saturated in dioxane, then the mixture was stirred for 1 h. After the reaction was completed, the solvent was removed by evaporation under reduced pressure and the residue was washed with acetone to obtain methyl (5-((4-(4-methylpiperazin-1-yl)phenyl)thio)-1H-benzo[d]imidazol-2-yl)carbamate hydrochloric acid salt (500 mg) as an off-white solid (yield 91%).

### 8.7.3. Method 3

Sodium chloride (NaCl) was added to a round-bottom flask. Concentrated sulfuric acid (c-H₂SO₄) was added dropwise. The resulting hydrogen chloride gas was injected through a tube into a suspension of methyl (5-((4-(4-methylpiperazin-1-yl)phenyl)thio)-1H-benzo[d]imidazol-2-yl)carbamate (Chemical Formula **6-1,** 40 mg, 0.1 mmol) in methanol (10 mL), creating an acidic solution. A precipitate was formed. Part of the solvent was removed, then ethyl ether was added and the precipitate was filtered to obtain methyl (5-((4-(4-methylpiperazin-1-yl)phenyl)thio)-1H-benzo[d]imidazol-2-yl)carbamate hydrochloric acid salt (30 mg) as a gray product (yield 69%).

### Example 9. Synthesis of Formula 6-2

The synthesis process of the thiobenzimidazole derivative of Chemical Formula 6-2 is shown by Reaction Scheme 9 presented below.

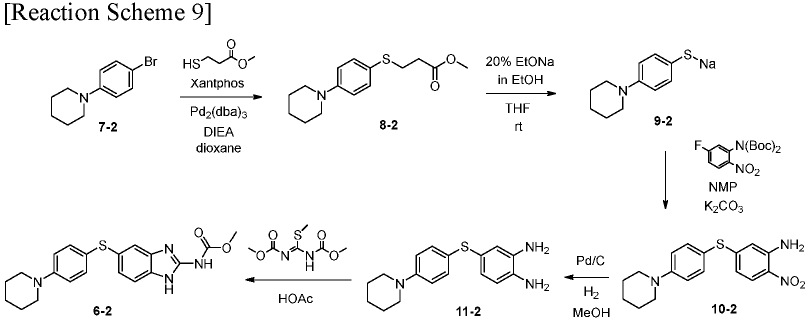

### 9.1. Synthesis of Methyl 3-((4-(piperidin-1-yl)phenyl)thio)propanoate (Chemical Formula 8-2)

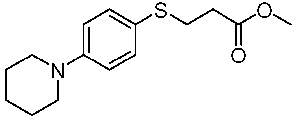

To a solution of 1-(4-bromophenyl)piperidine (Chemical Formula 7-2, 5.0 g, 20.83 mmol, 1.0 eq) in dry dioxane (50 mL), was added methyl 3-mercaptopropanoate (16.13 mL, 145.81 mmol, 7.0 eq), xantphos (2.4 g, 4.17 mmol, 0.2 eq). After the addition of N,N-Diisopropylethylamine (DIEA, 10.9 mL, 62.49 mmol, 3.0 eq) and Pd₂(dba)₃ (1.9 g, 2.08 mmol, 0.1 eq) was completed, the reaction mixture was stirred at 110°C under N₂ gas for 16 h. After the reaction was completed, the mixture was cooled to room temperature. The reaction mixture was filtered and the filtrate was concentrated. The residue was purified by silica gel column chromatography (HE: EtOAC = 40:1) to obtain 5.9 g of methyl 3-((4-(piperidin-1-yl)phenyl)thio)propanoate (Chemical Formula **8-2,** yield 100%) as a yellow oil.

¹H NMR (400 MHz, CDCl₃) δ 7.58 (d, J=8.6 Hz, 2H), 6.68 (d, J=8.6 Hz, 2H), 3.72 (s, 3H), 3.46 (m, 4H), 3.17(t, J =4.2 Hz, 2H), 2.60 (s, J = 4.2 Hz, 2H), 1.60-1.48 (m, 5H).

### 9.2. Synthesis of Sodium 4-(piperidin-1-yl)benzenethiolate (Chemical Formula 9-2)

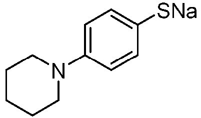

To a solution of methyl 3-((4-(piperidin-1-yl)phenyl)thio)propanoate (Chemical Formula **8-2, 3.6** g, 12.9 mmol, 1.0 eq) in dry THF (60 mL), was added 20% NaOEt (6.59 g, 19.355 mmol, 1.5 eq) at room temperature. The reaction mixture was heated to room temperature for 1 h. After the reaction was completed, the mixture was concentrated to obtain 3.45 g of the compound sodium 4-(piperidin-1-yl)benzenethiolate (Chemical Formula **9-2)** as a brown solid.

¹H NMR (400 MHz, CDCl₃) δ 7.22 (d, J=8.4 Hz, 2H), 6.65 (d, J=8.6 Hz, 2H), 3.47 (m, 4H), 1.58-1.90 (m, 5H).

### 9.3. Synthesis of 2-Nitro-5-((4-(piperidin-1-yl)phenyl)thio)aniline (Chemical Formula 10-2)

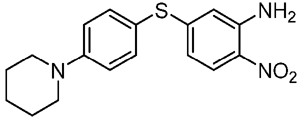

A solution of sodium 4-(piperidin-1-yl)benzenethiolate (Chemical Formula **9-2,** 3.45g, 16.05 mmol, 1.0 eq), 4-fluoro-2-di(tert-butoxycarbonyl)nitroaniline (5.98 g, 16.05 mmol, 1.0 eq) and K₂CO₃ (6.65 g, 48.15 mmol, 3.0 eq) in anhydrous 1-methyl-2-pyrrolidinone (NMP, 60 mL) was stirred at 130°C for 16 h under a nitrogen atmosphere. After the reaction was completed, the residue was purified by silica gel column chromatography (HE:EtOAC=20:1) to produce 0.630 g of 2-nitro-5-((4-(piperidin-1-yl)phenyl)thio)aniline (Chemical Formula **10-2)** as a yellow solid.

¹H NMR (400 MHz, DMSO-d₆) δ 7.82(d, J=6.8 Hz, 1H), 7.70 (s, 1H), 7.63 (d, J=6.8 Hz, 1H), 7.23 (d, J=8.8 Hz, 2H), 6.60 (d, J=8.8 Hz, 2H), 6.25 (br. s. 2H), 3.54 (m, 4H), 1.62-1.47 (m, 5H).

### 9.4. Synthesis of 4-((4-(Piperidin-1-yl)phenyl)thio)benzene-1,2-diamine (Chemical Formula 11-2)

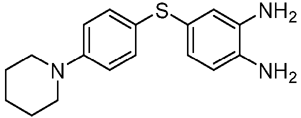

To a solution of 2-nitro-5-((4-(piperidin-1-yl)phenyl)thio)aniline (Chemical Formula **10-2,** 450 mg, 1.37 mmol, 1.0 eq) and Pd/C (110 mg) in MeOH (30 mL), was added MeOH/NH₃ (10 mL), and the solution was stirred at room temperature in a hydrogen (H₂) atmosphere under atmospheric pressure for 18 h. The reaction mixture was filtered and the filtrate was concentrated to obtain 0.370 g of 4-((4-(piperidin-1-yl)phenyl)thio)benzene-1,2-diamine (Chemical Formula **11-2,** yield 90.5%) as a purple solid.

¹H NMR (400 MHz, CDCl₃): δ ppm 7.21-7.25 (m, 2H), 6.85 (d, J = 6.8 Hz, 2H), 6.69-6.74 (m, 2H), 6.62 (d, J = 8.0 Hz, 1H), 3.34-3.43 (m, 4H), 3.14-3.16 (m, 4H), 1.55-1.69 (m, 6H)

### 9.5. Synthesis of (5-((4-(Piperidin-1-yl)phenyl)thio)-1H-benzo[d]imidazol-2-yl)carbamate (Chemical Formula 6-2)

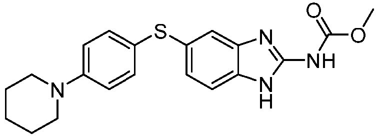

To a solution of 4-((4-(piperidin-1-yl)phenyl)thio)benzene-1,2-diamine (Chemical Formula **11-2,** 100 mg, 0.334 mmol, 1.0 eq) in acetic acid (5 mL), was added 1,3-bis(methoxycarbonyl)-2-methyl 2-thiopseudoeura (76 mg, 0.367 mmol, 1.1 eq), and the mixture was stirred at 80°C for 2 h. The reaction mixture was extracted with DCM and washed with saturated NaHCO₃. The organic layer was dried over anhydrous Na₂SO₄ and filtered. The filtrate was concentrated and the resulting mixture was purified by Prep-TLC to obtain 20 mg of methyl (5-((4-(piperidin-1-yl)phenyl)thio)-1H-benzo[d]imidazol-2-yl)carbamate (Chemical Formula **6-2,** yield 15.7%) as a white solid.
¹H NMR (400 MHz, DMSO-d6): δ 7.33(d, J = 8.4 Hz, 1H), 7.28 (d, J = 1.2 Hz, 1H), 7.20 (d, J = 8.8 Hz, 2H), 7.01-7.03 (m, 1H), 6.91 (d, J = 8.8 Hz, 2H), 6.08 (brs, 2H), 3.74 (s, 3H), 3.11-3.19 (m, 4H), 1.53-1.59 (m, 6H)

### Example 10. Synthesis of Chemical Formula 6-3

The synthesis process of the thiobenzimidazole derivative of Chemical Formula 6-3 is shown by Reaction Scheme 10 presented below.

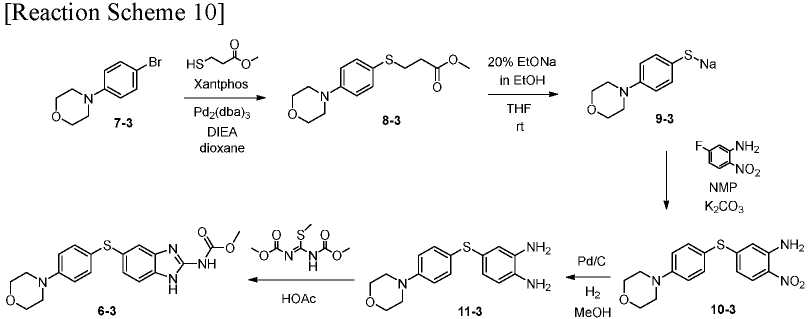

### 10.1. Synthesis of methyl 3-((4-morpholinophenyl)thio)propanoate (Chemical Formula 8-3)

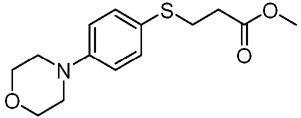

To a solution of 4-(4-bromophenyl)morpholine (Chemical Formula **7-3,** 7.0 g, 30.9 mmol, 1.0 eq) in dry dioxane (50 mL), were added methyl 3-mercaptopropanoate (18.6 g, 154.9 mmol, 5.0 eq) and xantphos (3.6 g, 6.2 mmol, 0.2 eq). After the addition of DIEA (12.2 g, 92.9 mmol, 3.0 eq) and Pd₂(dba)₃ (1.4 g, 1.6 mmol, 0.05 eq) was completed, the reaction mixture was stirred at 110°C for 16 h. After the reaction was completed, the mixture was cooled to room temperature. The reaction mixture was filtered and the filtrate was concentrated. The residue was purified by silica gel column chromatography (HE: EtOAC = 8:1) to obtain 7.5 g of methyl 3-((4-morpholinophenyl)thio)propanoate (Chemical Formula **8-3,** yield 91.2%) as a yellow oil.
¹H NMR (400 MHz, CDCl₃) δ 7.58 (d, J=8.8 Hz, 2H), 6.85 (d, J=8.6 Hz, 2H), 3.68 (s, 3H), 3.36 (m, 4H), 3.18 (m, 4H), 3.14 (t, J =4.2 Hz, 2H), 2.54 (t, J = 4.2 Hz, 2H).

### 10.2. Synthesis of sodium 4-morpholinobenzenethiolate (Chemical Formula 9-3)

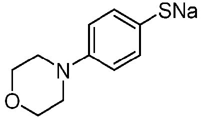

To a solution of methyl 3-((4-morpholinophenyl)thio)propanoate (Chemical Formula **8-3,** 7.4 g, 26.3 mmol, 1.0 eq) in dry THF (60 mL), was added 20% NaOEt (10.7 g, 31.6 mmol, 1.5 eq) at room temperature. The reaction mixture was heated to room temperature for 1 hour. After the reaction was completed, the mixture was concentrated to obtain 5.2 g of the compound sodium 4-morpholinobenzenethiolate (Chemical Formula **9-3,** yield 88.9%) as a brown oily liquid.

¹H NMR (400 MHz, CDCl₃) δ 7.25 (d, J=8.8 Hz, 2H), 6.60 (d, J=8.8 Hz, 2H), 3.65 (m, 4H), 3.20 (m, 4H).

### 10.3. Synthesis of 4-morpholino-2-nitroaniline (Chemical Formula 10-3)

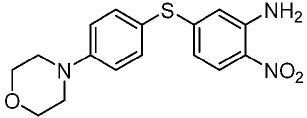

To a solution of 4-morpholinobenzenethiolate (Chemical Formula **9-3,** 5.2 g, 23.9 mmol, 1.0 eq) in anhydrous 1-methyl-2-pyrrolidinone (60 mL), were added 5-fluoro-2-nitroaniline (4.5 g, 28.7 mmol, 1.2 eq) and K₂CO₃ (9.9 g, 71.8 mmol, 3.0 eq) and the mixture was stirred at 130°C for 16 h under a nitrogen atmosphere. After the reaction was completed, the residue was purified by silica gel column chromatography (HE:EtOAC=20:1) to produce 1.2 g of 4-morpholino-2-nitroaniline (Chemical Formula **10-3,** yield 15.2%) as a yellow solid.

¹H NMR (400 MHz, DMSO-d6) δ 6.27 (br. s, 2H), 7.76 - 6.52 (m, 7H, aromatic H), 3.60 (m, 4H), 3.18 (m, 4H).

### 10.4. Synthesis of 4-morpholinobenzene-1,2-diamine (Chemical Formula 11-3)

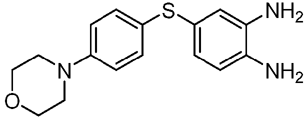

To a solution of 4-morpholino-2-nitroaniline (Chemical Formula **10-3,** 1.2 g, 3.6 mmol, 1.0 eq) in MeOH (30 mL), was added Pd/C (300 mg), and the solution was stirred at room temperature in the presence of hydrogen gas (H₂) for 18 h. The reaction mixture was filtered and the filtrate was concentrated to obtain 640 mg of 4-morpholinobenzene-1,2-diamine (Chemical Formula **11-3,** yield 60.1%) as a purple solid.
¹H NMR (400 MHz, DMSO-d₆): δ 7.85 (d, J = 9.2 Hz, 1H), 7.39-7.44 (m, 3H), 7.06 (d, J = 8.8 Hz, 2H), 6.52 (d, J = 1.6 Hz, 1H), 6.24-6.27 (m, 1H), 3.69 - 3.76 (m, 4H), 3.19-3.23 (m, 4H)

### 10.5. Synthesis of methyl (5-((4-morpholinophenyl)thio)-1H-benzo[d]imidazol-2-yl)carbamate (Chemical Formula 6-3)

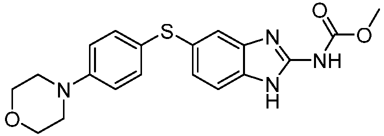

To a solution of 4-morpholinobenzene-1,2-diamine (Chemical Formula **11-3,** 100 mg, 0.332 mmol, 1.0 eq) in acetic acid (5 mL), was added 1,3-bis(methoxycarbonyl)-2-methyl 2-isothiourea (75 mg, 0.365 mmol, 1.1 eq.) and the mixture was stirred at 80°C for 2 h. It was confirmed by LCMS that the reaction was completed. The reaction mixture was extracted with DCM and washed with saturated sodium bicarbonate water, and the organic layer was dried over anhydrous Na₂SO₄ and filtered. The filtrate was concentrated and purified by Prep-TLC to obtain methyl (5-((4-morpholinophenyl)thio)-1H-benzo[d]imidazol-2-yl)carbamate (Chemical Formula **6-3,** yield 23.6%) as a white solid.
¹H NMR (400 MHz, DMSO-d₆): δ ppm 7.34 (d, J = 8.4 Hz, 1H), 7.30 (s, 1H), 7.23 (d, J = 8.8 Hz, 2H), 7.02-7.05 (m, 1H), 6.93 (d, J = 8.8 Hz, 2H), 3.74 (s, 3H), 3.71-3.73 (m, 4H), 3.10-3.12 (m, 4H)

### Example 11. Synthesis of Chemical Formula 6-4

The synthesis process of the thiobenzimidazole derivative of Chemical Formula 6-4 is shown by Reaction Scheme 11 presented below.

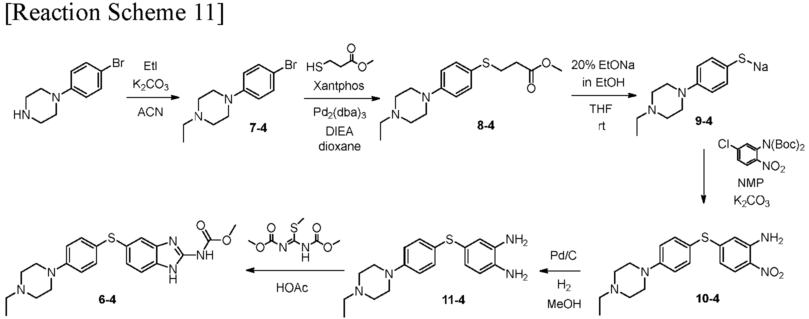

### 11.1. Synthesis of 1-(4-bromophenyl)-4-ethylpiperazine (Chemical Formula 7-4)

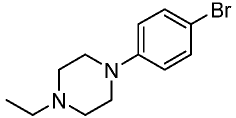

To a solution of 1-(4-bromophenyl)piperazine (10.0 g, 41.83 mmol, 1.0 eq) in anhydrous acetonitrile (50 mL), was added ethyl iodide (4.0 mL, 49.78 mmol, 1.2 eq) and K₂CO₃ (11.47 g, 82.96 mmol, 2.0 eq). After the addition was completed, the reaction mixture was stirred at 70°C for 2 h under nitrogen gas (N₂). The reaction mixture was filtered and the filtrate was concentrated. The residue was purified by silica gel (HE:EtOAC=5:1) column chromatography to obtain 9.84 g of 1-(4-bromophenyl)-4-ethylpiperazine (Chemical Formula **7-4,** yield 84.2%) as a white solid.

### 11.2. Synthesis of methyl 3-((4-(4-ethylpiperazin-1-yl)phenyl)thio)propanoate (Chemical Formula 8-4)

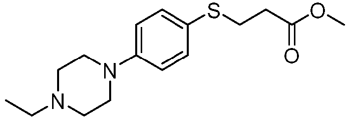

To a solution of 1-(4-bromophenyl)-4-ethylpiperazine (Chemical Formula **7-4,** 9.4 g, 34.9 mmol, 1.0 eq) in dry dioxane (50 mL), was added methyl 3-mercaptopropanoate (27.1 mL, 244.4 mmol, 7.0 eq) and xantphos (4.04 g, 6.98 mmol, 0.2 eq). After the addition of DIEA (18.3 mL, 104.7 mmol, 3.0 eq) and Pd₂(dba)₃ (3.2 g, 3.5 mmol, 0.1 eq) was completed, the reaction mixture was stirred at 110°C under an N₂ atmosphere for 16 h. The reaction mixture was filtered and the filtrate was concentrated. The residue was purified by silica gel column chromatography (Hexane: EtOAC = 2:1) to obtain methyl 3-((4-(4-ethylpiperazin-1-yl)phenyl)thio)propanoate (Chemical Formula **8-4,** yield 88.29%) as a yellow oil.

¹H NMR (400 MHz, CDCl₃) δ 7.68 (d, J=8.8 Hz, 2H), 6.67 (d, J=8.8 Hz, 2H), 3.65 (s, 3H), 3.64 - 3.44(m, 8H), 3.17 (t, J = 4.4 Hz, 2H), 2.50 (s, J = 4.4 Hz, 2H), 2.38 (q, J = 4.6 Hz, 2H), 1.02 (t, J = 4.6 Hz, 3H).

### 11.3. Synthesis of sodium 4-(4-ethylpiperazin-1-yl)benzenethiolate (Chemical Formula 9-4)

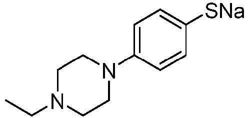

To a solution of 3-((4-(4-ethylpiperazin-1-yl)phenyl)thio)propanoate (Chemical Formula **8-4, 2.81** g, 9.1 mmol, 1.0 eq.) in dry THF (60 mL), was added 20% Sodium ethoxide (4.64 g, 13.65 mmol, 1.5 eq.), and then the reaction mixture was allowed to react at room temperature for 20 m. After the reaction was completed, the mixture was concentrated to obtain 2.8 g of sodium 4-(4-ethylpiperazin-1-yl)benzenethiolate (Chemical Formula **9-4)** as a brown solid.

¹H NMR (400 MHz, CDCl₃) δ 7.21 (d, J=8.6 Hz, 2H), 6.65 (d, J=8.6 Hz, 2H), 3.37 (m, 8H), 2.38 (q, J = 4.4 Hz, 2H), 1.10 (t, J = 4.4 Hz, 3H).

### 11.4. Synthesis of 5-((4-(4-ethylpiperazin-1-yl)phenyl)thio)-2-nitroaniline (Chemical Formula 10-4)

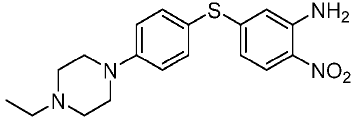

Sodium 4-(4-ethylpiperazin-1-yl)benzenethiolate (Chemical Formula **9-4,** 2.8 g, 11.5 mmol, 1.0 eq.) was dissolved in dry N-methylpyrrolidinedimethyl (60 mL). To the solution was sequentially added tert-butyl (tert-butoxycarbonyl)(5-chloro-2-nitrophenyl)carbamate (4.29 g, 11.5 mmol, 1.0 eq.) and K₂CO₃ (4.77 g, 34.5 mmol, 3.0 eq.), and the mixture was stirred at 130°C for 16 h under an N₂ atmosphere. After the reaction was completed, the residue was purified by silica gel column chromatography (DCM:MeOH = 20:1) to obtain 520 mg of 5-((4-(4-ethylpiperazin-1-yl)phenyl)thio)-2-nitroaniline (Chemical Formula **10-4)** as an orange solid.

¹H NMR (400 MHz, DMSO-d₆) δ 7.88- 6.50 (m, 7H, aromatic H), 6.24 (br. s, 2H), 3.64 (s, 8H), 2.34 (q, J = 4.4 Hz, 2H), 1.10 (t, J = 4.4 Hz, 3H).

### 11.5. Synthesis of 4-((4-(4-ethylpiperazin-1-yl)phenyl)thio)benzene-1,2-diamine (Chemical Formula 11-4)

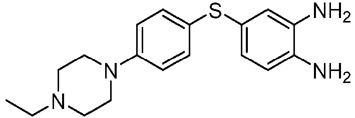

To a solution of 5-((4-(4-ethylpiperazin-1-yl)phenyl)thio)-2-nitroaniline (Chemical Formula **10-4,** 520 mg, 1.45 mmol) in MeOH (15 mL) and MeOH/NH₃ (5 mL), was added Pd/C (150 mg), and the mixture was stirred in the presence of hydrogen gas (H₂) at room temperature for 18 h. The reaction mixture was filtered and the filtrate was concentrated to obtain 470 mg of 4-((4-(4-ethylpiperazin-1-yl)phenyl)thio)benzene-1,2-diamine (Chemical Formula **11-4,** yield 99.5%) as a purple solid.
¹H NMR (400 MHz, CDCl₃): δ 7.22 (d, *J* = 8.4 Hz, 2H), 6.82 (d, *J =* 8.8 Hz, 2H), 6.73-6.78 (m, 2H), 6.64 (d, *J* = 7.6 Hz, 1H), 3.26-3.46 (m, 8H), 2.78-2.92 (m, 4H), 1.51-1.64 (m, 2H), 1.29-1.30 (m, 3H)

### 11.6. Synthesis of Methyl (5-((4-(4-ethylpiperazin-1-yl)phenyl)thio)-1H-benzo[d]imidazol-2-yl)carbamate (Chemical Formula 6-4)

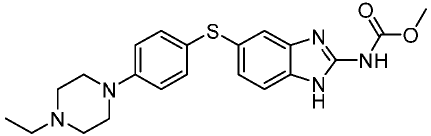

To a solution of 4-((4-(4-ethylpiperazin-1-yl)phenyl)thio)benzene-1,2-diamine (Formula **11-4,** 100 mg, 0.305 mmol, 1.0 eq) in acetic acid (5 mL), was added 1,3-bis(methoxycarbonyl)-2-methyl 2-isothiourea (69 mg, 0.335 mmol, 1.1 eq.) and the mixture was stirred at 80°C for 2 h. It was confirmed by LCMS that the reaction was completed. The reaction mixture was extracted with DCM and washed with saturated NaHCO₃. The filtrate was concentrated and purified by Prep-TLC to obtain 44 mg of methyl (5-((4-(4-ethylpiperazin-1-yl)phenyl)thio)-1H-benzo[d]imidazol-2-yl)carbamate (Chemical Formula **6-4, 44** mg) as a white solid.
¹H NMR (400 MHz, DMSO-d₆) δ 7.33 (d, J = 8.0 Hz, 1H), 7.29 (s, 1H), 7.21 (d, J = 8.8 Hz, 2H), 7.00-7.03 (m, 1H), 6.92 (d, J = 8.8 Hz, 2H), 3.74 (s, 3H), 3.13-3.15 (m, 4H), 2.46-2.48 (m, 4H), 2.32-2.37 (m, 2H), 1.01-1.04 (m, 3H)

### Example 12. Synthesis of Chemical Formula 6-5

The synthesis process of the thiobenzimidazole derivative of Chemical Formula 6-5 is shown by Reaction Scheme 12 presented below.

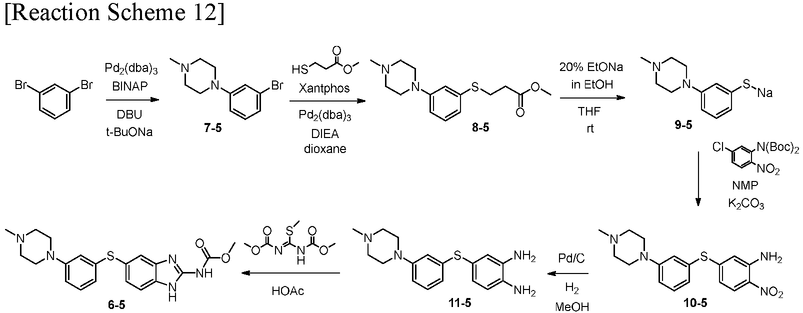

### 12.1. Synthesis of 1-(3-bromophenyl)-4-methylpiperazine (Chemical Formula 7-5)

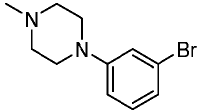

To a solution of 1,3-dibromobenzene (9.0 g, 38.5 mmol, 1.0 eq) in toluene (100 mL), were added 1-methylpiperazine (19.2 g, 192.4 mmol, 5.0 eq), BINAP (4.8 g, 7.7 mmol, 0.2 eq), DBU (17.5 g, 115.5 mmol, 3.0 eq) and Pd₂(dba)₃ (2.2 g, 1.9 mmol, 0.05 eq). The reaction mixture was heated to 60°C. Freshly ground sodium tert-butoxide (12.9 g, 192.4 mmol, 3.0 eq) was added. The reaction mixture was stirred at 60°C under an N₂ atmosphere for 16 h, then the mixture was concentrated. The residue was separated and purified by silica gel column chromatography (HE:EtOAC=60:1) to produce 5.5 g of 1-(3-bromophenyl)-4-methylpiperazine (Chemical Formula **7-5,** yield 56.3%) as a yellow oil.

### 12.2. Synthesis of methyl 3-((3-(4-methylpiperazin-1-yl)phenyl)thio)propanoate (Chemical Formula 8-5)

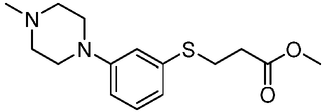

To a solution of 1-(3-bromophenyl)-4-methylpiperazine (Chemical Formula **7-5,** 5.5 g, 21.65 mmol, 1.0 eq) in dry dioxane (50 mL), was added methyl 3-mercaptopropanoate (18.2 mg, 155.55 mmol, 7.0 eq). To the mixture were sequentially added xantphos (2.5 g, 4.33 mmol, 0.2 eq), DIEA (88.4 g, 64.95 mmol, 3.0 eq) and Pd₂(dba)₃ (2.0 g, 2.165 mmol, 0.1 eq). The reaction mixture was stirred at 110°C under an N₂ atmosphere for 24 h. The reaction mixture was filtered and the filtrate was concentrated. The residue was purified by silica gel column chromatography (DCM:MeOH=20:1) to obtain 4.5 g of methyl 3-((3-(4-methylpiperazin-1-yl)phenyl)thio)propanoate (Chemical Formula **8-5,** yield 70.7%) as a yellow oil.

¹H NMR (400 MHz, CDCl₃) δ 7.40 (s, 1H), 7.28 (d, J=8.6 Hz, 1H), 6.75 (d, J=8.6 Hz, 1H), 3.66 (s, 3H), 3.45 (s, 4H), 3.15 (t, J =4.2 Hz, 2H), 2.58 (s, J = 4.2 Hz, 2H), 2.33 (s, 4H), 2.23 (s, 3H).

### 12.3. Synthesis of sodium 3-(4-methylpiperazin-1-yl)benzenethiolate (Chemical Formula 9-5)

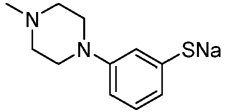

To a solution of methyl 3-((3-(4-(piperidin-1-yl)phenyl)thio)propanoate (Chemical Formula **8-5,** 2.3 g, 7.82 mmol, 1.0 eq) in dry THF (60 mL), was added 20% NaOEt (3.8 g, 11.73 mmol, 1.5 eq). The reaction mixture was heated at room temperature for 1 h. After the reaction was completed, the mixture was concentrated to obtain 2.05 g of sodium 3-(4-(methylpiperazin-1-yl)benzenethiolate (Chemical Formula 9-5) as a brown solid.

¹H NMR (400 MHz, CDCl₃) δ 7.36 (s, 1H), 7.24 (d, J=8.4 Hz, 1H), 6.55 (d, J=8.6 Hz, 1H), 3.44 (s, 4H), 2.38 (s, 4H), 2.23 (s, 3H).

### 12.4. Synthesis of 5-((3-(4-methylpiperazin-1-yl)phenyl)thio)-2-nitroaniline (Chemical Formula 10-5)

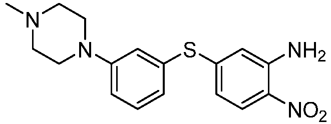

To a solution of sodium 3-(4-methylpiperazin-1-yl)benzenethiolate (Chemical Formula 9-5, 2.05 g, 8.91 mmol, 1.0 eq.) in dry N-methylpyrrolidinedimethyl (60 mL), were sequentially added tert-butyl (tert-butoxycarbonyl)(5-chloro-2-nitrophenyl)carbamate (3.3 g, 8.91 mmol, 1.0 eq.) and K₂CO₃ (3.7 g, 26.73 mmol, 3.0 eq.), and the reaction mixture was stirred at 130°C for 16 h under an N₂ atmosphere. After the reaction was completed, the residue was purified by silica gel column chromatography (DCM:MeOH = 20:1) to obtain 3.1 g of 5-((3-(4-methylpiperazin-1-yl)phenyl)thio)-2-nitroaniline (Chemical Formula **10-5)** as an orange solid.

¹H NMR (400 MHz, DMSO-d6) δ 7.84 - 6.58 (m, 7H, aromatic H), 6.43 (br. s, 2H), 3.44 (s, 4H), 2.36 (s, 4H), 2.23 (s, 3H).

### 12.5. Synthesis of 4-((3-(4-methylpiperazin-1-yl)phenyl)thio)benzene-1,2-diamine (Chemical Formula 11-5)

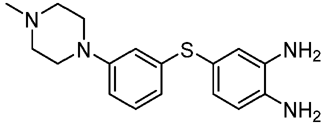

To a solution of 5-((3-(4-methylpiperazin-1-yl)phenyl)thio)-2-nitroaniline (Chemical Formula 10-5, 3.1 g, 9.0 mmol, 1.0 eq) and Pd/C (1.0 g) in MeOH solution (60 mL), was added MeOH/NH₃ (20 mL) and the mixture was stirred in the presence of H₂ for 18 h. The reaction mixture was filtered, then the filtrate was concentrated to obtain 600 mg of 4-((3-(4-methylpiperazin-1-yl)phenyl)thio)benzene-1,2-diamine (Chemical Formula **11-5)** as a gray solid.

¹H NMR (400 MHz, CDCl₃): δ 7.07-7.12 (m, 1H), 6.84-6.89 (m, 2H), 6.78-6.80 (m, 1H), 6.66-6.70 (m, 2H), 6.60-6.63 (m, 1H), 3.49 (s, 2H), 3.36 (s, 2H), 3.14-3.17 (m, 4H), 2.52-2.55 (m,4H), 2.34(d, J= 5.6 Hz,3H)

### 12.6. Synthesis of methyl (5-((3-(4-methylpiperazin-1-yl)phenyl)thio)-1H-benzo[d]imidazol-2-yl)carbamate (Chemical Formula 6-5)

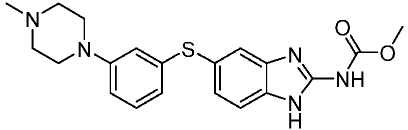

To a solution of 4-((3-(4-Methylpiperazin-1-yl)phenyl)thio)benzene-1,2-diamine (Chemical Formula **11-5,** 100 mg, 0.318 mmol, 1.0 eq) in acetic acid (CH₃COOH, 10 mL), was added 1,3-bis(methoxycarbonyl)-2-methyl 2-isothiourea (72 mg, 0.35 mmol, 1.1 eq.). The reaction mixture was stirred at 80°C for 2 h. It was confirmed by LCMS that the reaction was completed. The reaction mixture was extracted with DCM and washed with saturated NaHCO₃. The organic layer was dried with anhydrous Na₂SO₄ and filtered. The filtrate was concentrated and the mixture was purified by Prep-TLC to obtain 20 mg of methyl (5-((3-(4-methylpiperazin-1-yl)phenyl)thio)-1H-benzo[d]imidazol-2-yl)carbamate (Chemical Formula 6-5, yield 15.87%) as a white solid.
¹H NMR (400 MHz, DMSO-d₆) δ 7.49 (s, 1H), 7.43 (d, J= 8.0 Hz, 1H), 7.16-7.18 (m, 1H), 7.08-7.12 (m, 1H), 6.77 (d, J= 6 Hz, 2H), 6.47 (d, J=7.6 Hz, 1H), 3.76 (s, 3H), 3.04-3.07 (m, 4H), 2.38-2.40 (m, 4H), 2.19 (s, 3H)

### Example 13. Synthesis of Chemical Formula 6-6

The synthesis process of the thiobenzimidazole derivative of Chemical Formula 6-6 is shown by Reaction Scheme 13 presented below.

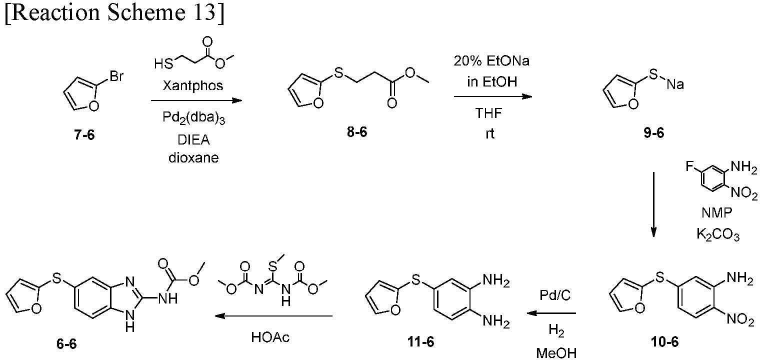

### 13.1. Synthesis of methyl 3-(furan-2-ylthio)propanoate (Chemical Formula 8-6)

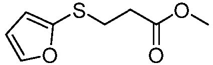

To a solution of 2-Bromofuran (Chemical Formula **7-6, 6.0** g, 40.8 mmol, 1.0 eq) in dry dioxane (50 mL), was sequentially added methyl 3-mercaptopropanoate (31.6 mL, 285.6 mmol, 7.0 eq), Xantphos (4.787 g, 8.16 mmol, 0.2 eq), DIEA (21.4 mL, 122.4 mmol, 3.0 eq) and Pd₂(dba)₃ (3.623 g, 4.08 mmol, 0.1 eq). After the addition was completed, the reaction mixture was stirred at 110°C under N₂ gas for 16 h. The reaction mixture was filtered and the filtrate was concentrated. The resulting product was purified by silica gel column chromatography (HE: EtOAC = 40:1) to obtain 6.34 g of methyl 3-(furan-2-ylthio)propanoate (Chemical Formula **8-6,** yield 83.4%) as a yellow oily liquid.

### 13.2. Synthesis of sodium furan-2-thiolate (Chemical Formula 9-6)

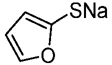

To a solution of methyl 3-(furan-2-ylthio)propanoate (Chemical Formula **8-6,** 6.34 g, 34.08 mmol, 1.0 eq) in dry THF (60 mL), was added 20% NaOEt (14.06 g, 41.32 mmol, 1.2 eq) at room temperature. The reaction mixture was heated for 1 h. After the reaction was completed, the mixture was concentrated to obtain 4.61 g of sodium furan-2-thiolate (Chemical Formula **9-6)** as a brown solid.

¹H NMR (400 MHz, CDCl₃) δ 7.75 (d, J=6.4 Hz, 1H), 7.13 (d, J=6.4 Hz, 1H), 6.55 (m, 1H).

### 13.3. Synthesis of 5-(furan-2-ylthio)-2-nitroaniline (Chemical Formula 10-6)

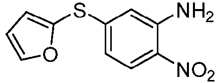

A solution of sodium furan-2-thiolate (Chemical Formula **9-6,** 4.61 g, 37.86 mmol, 1.1 eq), 5-fluoro-2-nitroaniline (5.39 g, 34.4 mmol, 1.0 eq) and K₂CO₃ (14.26 g, 103.2 mmol, 3.0 eq) ) in an anhydrous N-methylpyrrolidinedimethyl (60 mL) was stirred at 130°C for 16 h under an N₂ atmosphere. After the reaction was completed, the residue was purified by silica gel column chromatography (HE: EtOAC = 20: 1) to obtain 860 mg of 5-(furan-2-ylthio)-2-nitroaniline (Chemical Formula **10-6)** as a yellow solid.

¹H NMR (400 MHz, DMSO-d₆) δ 7.85 - 6.48 (m, 6H, aromatic H), 6.35 (br, s, 2H).

### 13.4. Synthesis of 4-(furan-2-ylthio)benzene-1,2-diamine (Chemical Formula 11-6)

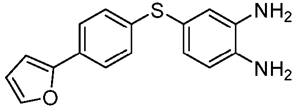

To a mixed solution of MeOH (36 mL) and MeOH/NH₃ (12 mL), was added 5-(furan-2-ylthio)-2-nitroaniline (Chemical Formula **10-6,** 860 mg, 3.624 mmol, 1.0 eq) and Pd/C (360 mg) and the mixture was stirred in the presence of H₂ at room temperature for 18 h. The reaction mixture was filtered and the filtrate concentrated to obtain 750 mg of 4-(furan-2-ylthio)benzene-1,2-diamine (Chemical Formula **11-6,** yield 100%) as a purple solid.

¹H NMR (400 MHz, CDCl₃): δ ppm 7.49-7.50 (m, 1H), 6.69-6.74 (m, 2H), 6.59-6.62 (m, 2H), 6.39-6.41 (m, 1H), 3.37 (brs, 4H)

### 13.5. Synthesis of methyl (5-(furan-2-ylthio)-1H-benzo[d]imidazol-2-yl)carbamate (Chemical Formula 6-6)

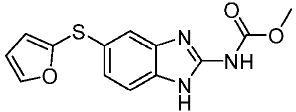

To a solution of 4-(furan-2-ylthio)benzene-1,2-diamine (Chemical Formula **11-6,** 100 mg, 0.485 mmol, 1.0 eq) in acetic acid (CH₃COOH, 5 mL), was added 1,3-bis(methoxycarbonyl)-2-methyl 2-isothiourea (100 mg, 0.485 mmol, 1.1 eq.). The reaction mixture was stirred at 80°C for 2 h. It was confirmed by LCMS that the reaction was completed. The reaction mixture was extracted with DCM and washed with saturated NaHCO₃. The organic layer was dried with anhydrous Na₂SO₄ and filtered. The filtrate was concentrated and the reaction residue was purified by Prep-TLC to obtain 20 mg of methyl (5-(furan-2-ylthio)-1H-benzo[d]imidazol-2-yl)carbamate (Chemical Formula **6-6,** 20 mg, yield 14.2%) as a white solid.
¹H NMR (400 MHz, DMSO-d₆): δ ppm 7.88-7.89 (m, 1H), 7.40 (d, J = 8.4 Hz, 1H), 7.30 (d, J = 8.4 Hz, 1H), 7.07-7.10 (m, 1H), 6.92-6.93 (m, 1H), 6.61-6.63 (m, 1H), 3.78 (s, 3H)

While embodiments are described, it will be apparent to one of ordinary skill in the art that various alterations and modifications in form and details may be made to these embodiments without departing from the spirit and scope of the claims and their equivalents. For example, suitable results may be achieved if the described techniques are performed in a different order, and/or if components in a described system, architecture, device, or circuit are combined in a different manner, or replaced or supplemented by other components or their equivalents.

Therefore, other implementations, other embodiments, and equivalents to the claims are also within the scope of the following claims.

## Claims

1. A method for preparing a thiobenzimidazole derivative represented by [Chemical Formula 1], the method comprising the following steps:(1) preparing a compound represented by [Chemical Formula 3] by adding potassium O-ethylcarbonodithioate to a compound represented by [Chemical Formula 2];
(2) preparing a compound represented by [Chemical Formula 4] by adding 5-chloro-2-nitroaniline to the compound represented by [Chemical Formula 3] as prepared above; and
(3) preparing a compound represented by [Chemical Formula 1] by adding 1,3-bis(methoxycarbonyl)-2-methyl 2-thiopseudoeura to the compound represented by [Chemical Formula 4] as prepared above. 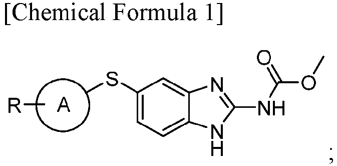 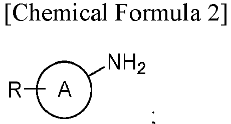 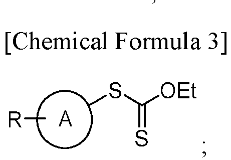 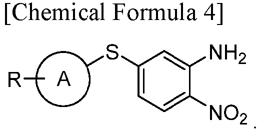 (in Chemical Formulae 1 to 4,
A is a C₃-C₁₀ cyclic alkyl group, a heterocycloalkyl group, an aryl group, or a heteroaryl group, and
**R** is one or more selected from the group consisting of hydrogen, a halogen group, a chained or cyclic alkyl group of C₁-C₁₀, a heterocycloalkyl group, an aryl group, and a heteroaryl group,
wherein the cyclic alkyl group, the heterocycloalkyl group, the aryl group, or the heteroaryl group is unsubstituted or substituted with one or more selected from the group consisting of a C₁-C₁₀ chained alkyl group, an alkylsulfonyl group, and a carbonyl group, and
wherein the carbonyl group is substitutable with a chained or cyclic alkyl group of C₁-C₁₀, a heterocycloalkyl group, an aryl group, or a heteroaryl group.)

2. The method of claim 1, wherein the compound represented by [Chemical Formula 1] is any one selected from the group consisting of the following [Chemical Formula 1-1] to [Chemical Formula 1-7]: 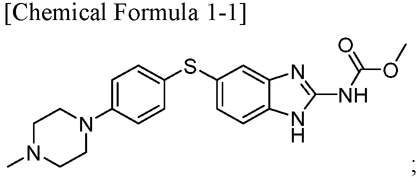 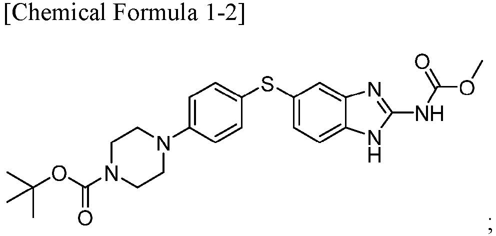 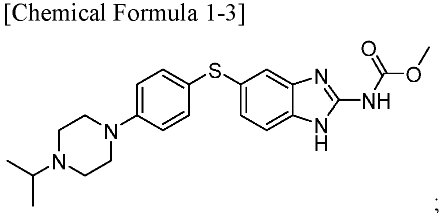 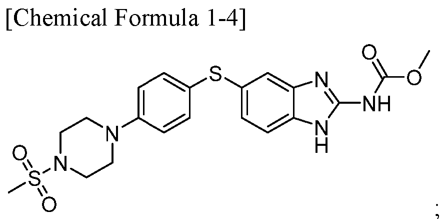 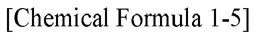 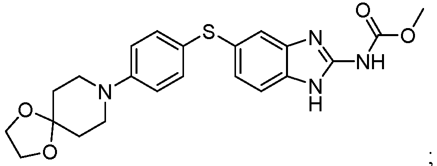 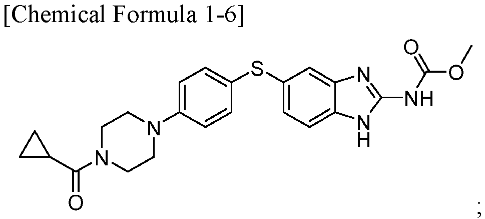 and

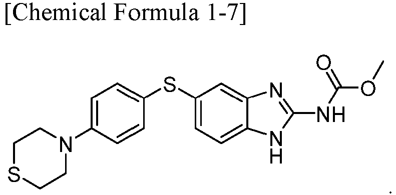

3. The method of claim 1, wherein the compound represented by [Chemical Formula 2] is any one selected from the group consisting of the following [Chemical Formula 2-1] to [Chemical Formula 2-7]: 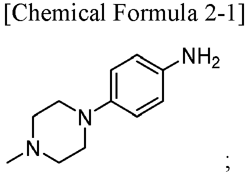 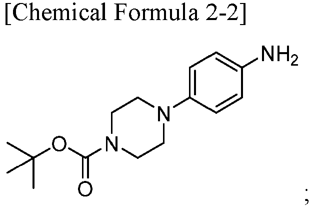 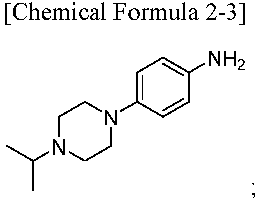 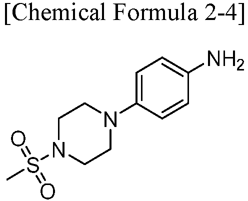 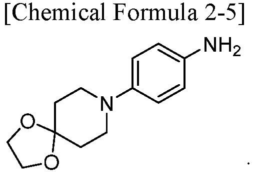 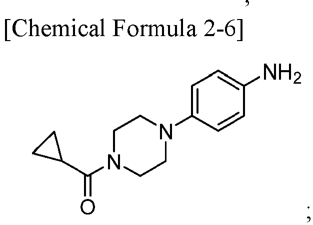 and

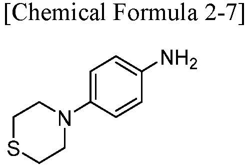

4. The method of claim 1, wherein the compound represented by [Chemical Formula 3] is any one selected from the group consisting of the following [Chemical Formula 3-1] to [Chemical Formula 3-7]: 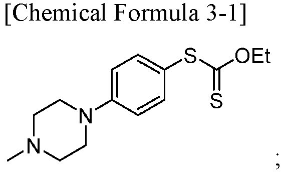 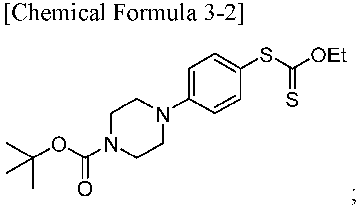 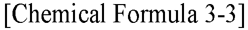 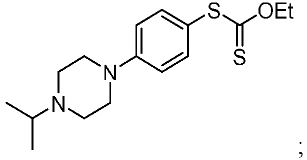 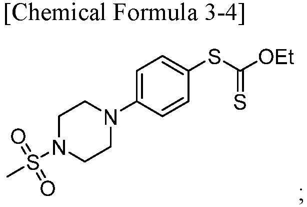 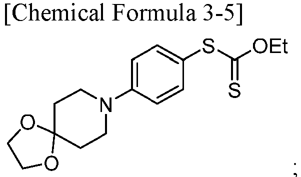 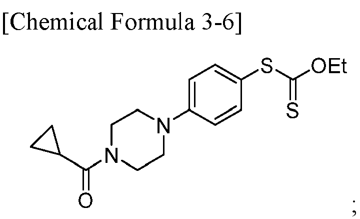 and

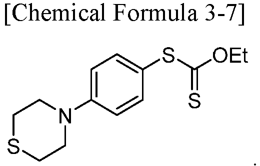

5. The method of claim 1, wherein the compound represented by [Chemical Formula 4] is any one selected from the group consisting of the following [Chemical Formula 4-1] to [Chemical Formula 4-7]: 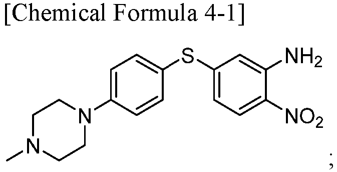 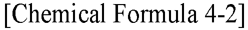 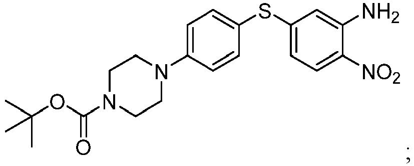 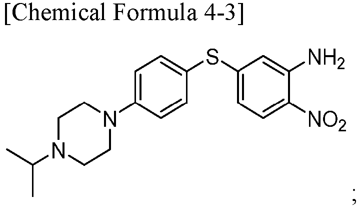 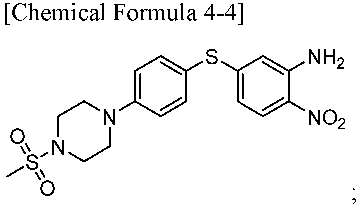 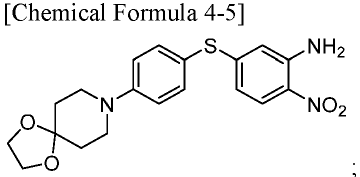 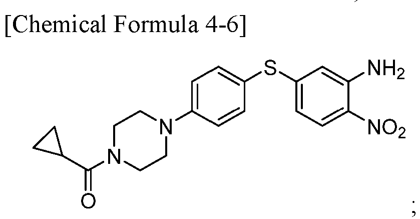 and

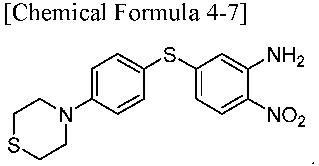

6. The method of claim 1, wherein the step (1) is **characterized by** adding sulfuric acid (H₂SO₄) and sodium nitrite (NaNO₂) and performing stirring, and then adding potassium O-ethylcarbonodithioate.

7. The method of claim 1, wherein the step (2) is **characterized by** adding sodium methoxide (MeONa) or methanol (MeOH) together with 5-chloro-2-nitroaniline to prepare the compound represented by [Chemical Formula 4] without reduction.

8. The method of claim 1, wherein the step (3) is **characterized by** adding acetic acid (AcOH) and zinc (Zn) together with 1,3-bis(methoxycarbonyl)-2-methyl 2-thiopseudoeura.

9. The method of claim 1, wherein the step (3) is **characterized by** synthesizing the following [Chemical Formula 5] from the compound represented by [Chemical Formula 4], and then adding 1,3-bis(methoxycarbonyl)-2-methyl 2-thiopseudoeura to prepare the compound represented by [Chemical Formula 1]. 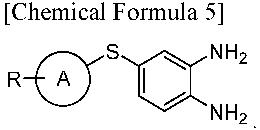 (in Chemical Formula 5,
A is a C₃-C₁₀ cyclic alkyl group, a heterocycloalkyl group, an aryl group, or a heteroaryl group, and
R is one or more selected from the group consisting of hydrogen, a halogen group, a chained or cyclic alkyl group of C₁-C₁₀, a heterocycloalkyl group, an aryl group, and a heteroaryl group, wherein the cyclic alkyl group, the heterocycloalkyl group, the aryl group, or the heteroaryl group is unsubstituted or substituted with one or more selected from the group consisting of a C₁-C₁₀ chained alkyl group, an alkylsulfonyl group, and a carbonyl group, and
wherein the carbonyl group is substitutable with a chained or cyclic alkyl group of C₁-C₁₀, a heterocycloalkyl group, an aryl group, or a heteroaryl group.

10. The method of claim 9, wherein a compound represented by [Chemical Formula 5] is any one selected from the group consisting of the following [Chemical Formula 5-1] and [Chemical Formula 5-3] to [Chemical Formula 5-7]: 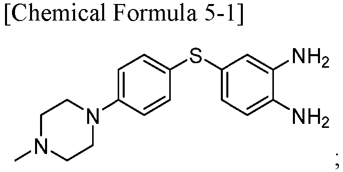 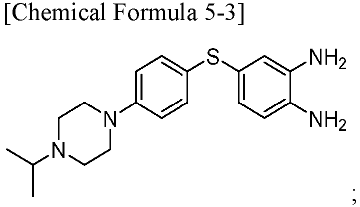 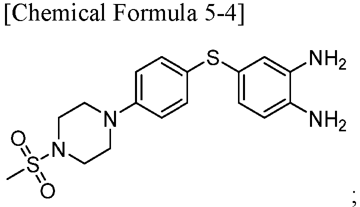 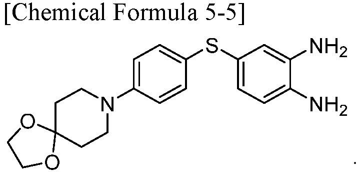 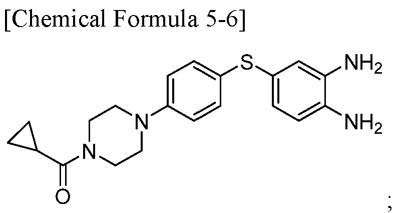 and

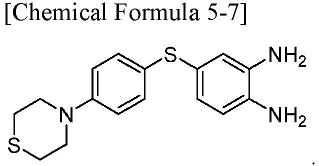

11. The method of claim 9, wherein the step (3) is **characterized by** adding acetic acid (AcOH) and zinc (Zn) to the compound represented by [Chemical Formula 4] to synthesize the compound represented by [Chemical Formula 5].

12. The method of claim 9, wherein the step (3) is **characterized by** adding methanol (MeOH) and palladium/carbon (Pd/C) to the compound represented by [Chemical Formula 4] to synthesize the compound represented by [Chemical Formula 5].

13. The method of claim 9, wherein the step (3) is **characterized by** adding ethanol (EtOH) and tin chloride (SnCl₂) to the compound represented by [Chemical Formula 4] to synthesize the compound represented by [Chemical Formula 5].

14. A method for preparing a pharmaceutically acceptable salt of a thiobenzimidazole derivative represented by [Chemical Formula 1], the method comprising the following steps:
(1) preparing a compound represented by [Chemical Formula 3] by adding potassium O-ethylcarbonodithioate to a compound represented by [Chemical Formula 2];
(2) preparing a compound represented by [Chemical Formula 4] by adding 5-chloro-2-nitroaniline to the compound represented by [Chemical Formula 3] as prepared above;
(3) preparing a compound represented by [Chemical Formula 1] by adding 1,3-bis(methoxycarbonyl)-2-methyl 2-thiopseudoeura to the compound represented by [Chemical Formula 4] as prepared above; and
(4) preparing a hydrogen chloride (HCl) salt of the compound represented by [Chemical Formula 1] by adding HCL to the compound represented by [Chemical Formula 1] as prepared above. 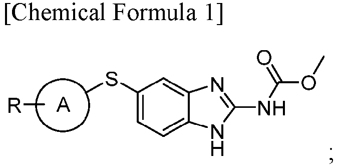 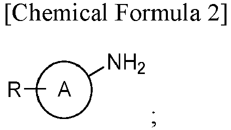 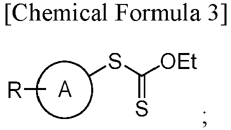 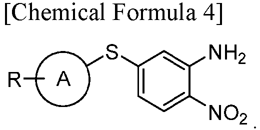 (in Chemical Formulae 1 to 4,
A is a C₃-C₁₀ cyclic alkyl group, a heterocycloalkyl group, an aryl group,
or a heteroaryl group, and
R is one or more selected from the group consisting of hydrogen, a halogen group, a chained or cyclic alkyl group of C₁-C₁₀, a heterocycloalkyl group, an aryl group, and a heteroaryl group,
wherein the cyclic alkyl group, the heterocycloalkyl group, the aryl group, or the heteroaryl group is unsubstituted or substituted with one or more selected from the group consisting of a C₁-C₁₀ chained alkyl group, an alkylsulfonyl group, and a carbonyl group, and
wherein the carbonyl group is substitutable with a chained or cyclic alkyl group of C₁-C₁₀, a heterocycloalkyl group, an aryl group, or a heteroaryl group.)

15. The method of claim 14, wherein the step (4) is **characterized by** adding methanol (MeOH) and hydrogen chloride (HCl) to the compound represented by [Chemical Formula 1], and then sequentially adding isopropyl alcohol and isopropyl ether to prepare the HCl salt of the compound represented by [Chemical Formula 1].

16. The method of claim 14, wherein the step (4) is **characterized by** adding methylene chloride (CH₂Cl₂) and methanol (MeOH) to the compound represented by [Chemical Formula 1] and performing cooling, and then adding hydrogen chloride (HCl) saturated in dioxane to prepare the HCl salt of the compound represented by [Chemical Formula 1].

17. The method of claim 14, wherein the step (4) is **characterized by** adding hydrogen chloride (HCl) gas to the compound represented by [Chemical Formula 1], and then adding ethyl ether to prepare the HCl salt of the compound represented by [Chemical Formula 1].

18. A method for preparing a thiobenzimidazole derivative represented by [Chemical Formula 6], the method comprising the following steps:
(1) preparing a compound represented by [Chemical Formula 8] by adding methyl 3-mercaptopropanoate, Xantphos, DIEA (N,N-diisopropylethylamine), and Pd₂(dba)₃ to a compound represented by [Chemical Formula 7];
(2) preparing a compound represented by [Chemical Formula 9] by adding sodium ethoxide (EtONa) to the compound represented by [Chemical Formula 8] as prepared above;
(3) preparing a compound represented by [Chemical Formula 10] by adding one or more selected from the group consisting of 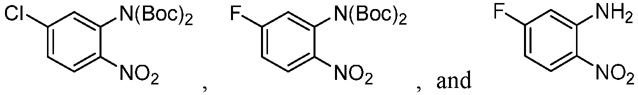 to the compound represented by [Chemical Formula 9] as prepared above; and
(4) preparing a compound represented by [Chemical Formula 6] by adding 1,3-bis(methoxycarbonyl)-2-methyl 2-thiopseudoeura to the compound represented by [Chemical Formula 10] as prepared above. 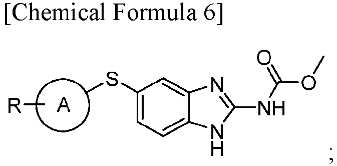 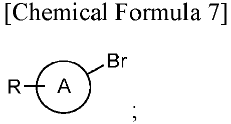 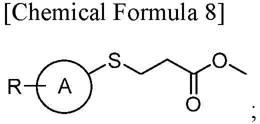 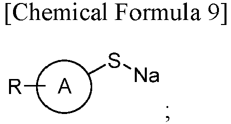 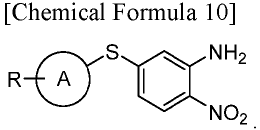 (in Chemical Formulae 6 to 10,
A is a C₃-C₁₀ cyclic alkyl group, a heterocycloalkyl group, an aryl group, or a heteroaryl group, and
R is one or more selected from the group consisting of hydrogen, a halogen group, a chained or cyclic alkyl group of C₁-C₁₀, a heterocycloalkyl group, an aryl group, and a heteroaryl group,
wherein the cyclic alkyl group, the heterocycloalkyl group, the aryl group, or the heteroaryl group is unsubstituted or substituted with one or more selected from the group consisting of a C₁-C₁₀ chained alkyl group, an alkylsulfonyl group, and a carbonyl group, and
wherein the carbonyl group is substitutable with a chained or cyclic alkyl group of C₁-C₁₀, a heterocycloalkyl group, an aryl group, or a heteroaryl group.)

19. The method of claim 18, wherein the compound represented by [Chemical Formula 6] is any one selected from the group consisting of the following [Chemical Formula 6-1] to [Chemical Formula 6-6]: 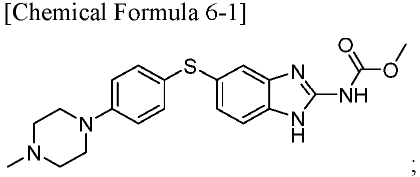 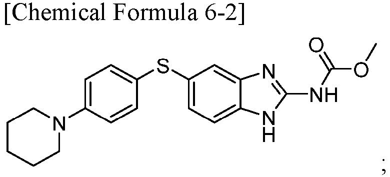 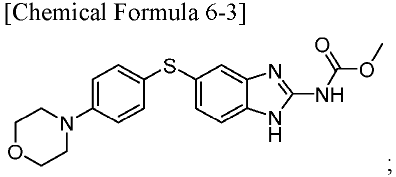 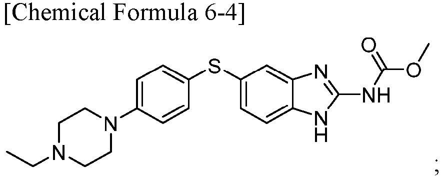 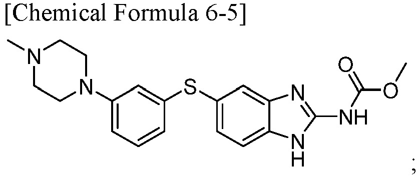 and

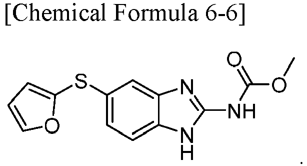

20. The method of claim 18, wherein the compound represented by [Chemical Formula 7] is any one selected from the group consisting of the following [Chemical Formula 7-1] to [Chemical Formula 7-6]: 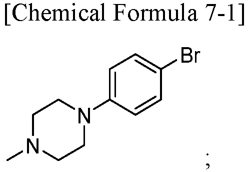 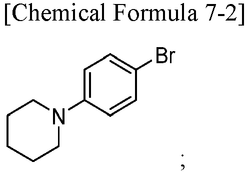 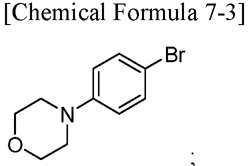 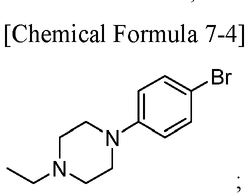 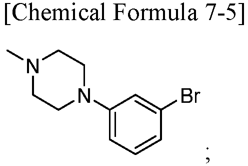 and

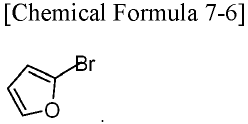

21. The method of claim 18, wherein the compound represented by [Chemical Formula 8] is any one selected from the group consisting of the following [Chemical Formula 8-1] to [Chemical Formula 8-6]: 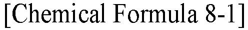 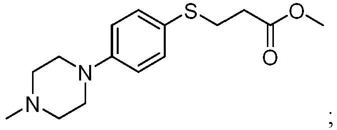 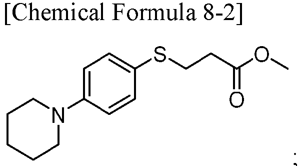 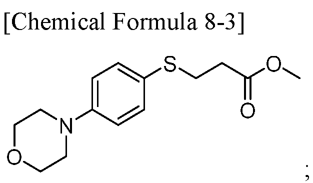 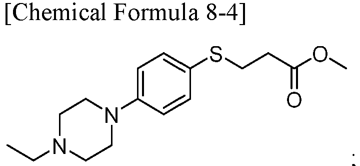 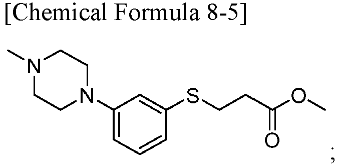 and

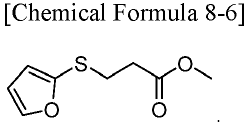

22. The method of claim 18, wherein the compound represented by [Chemical Formula 9] is any one selected from the group consisting of the following [Chemical Formula 9-1] to [Chemical Formula 9-6]: 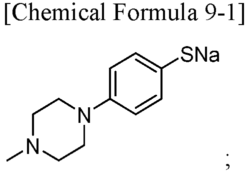 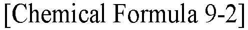 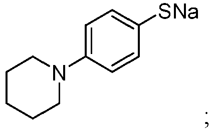 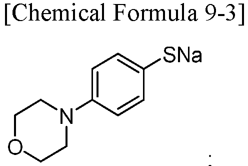 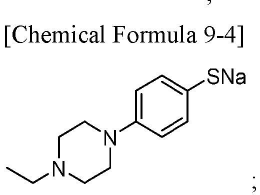 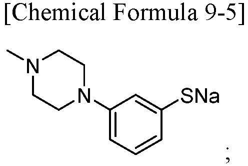 and

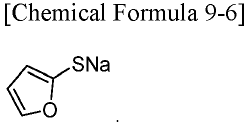

23. The method of claim 18, wherein the compound represented by [Chemical Formula 10] is any one selected from the group consisting of the following [Chemical Formula 10-1] to [Chemical Formula 10-6]: 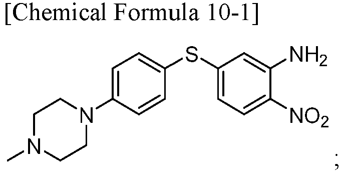 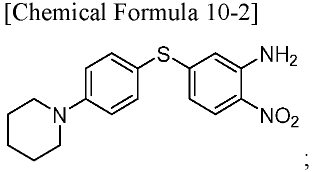 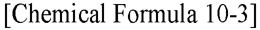 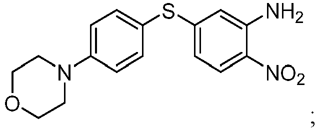 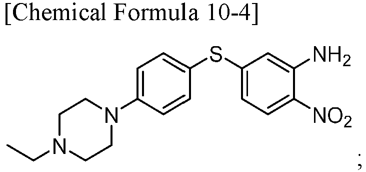 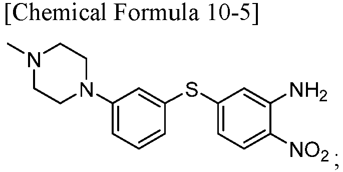 and

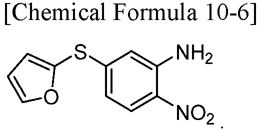

24. The method of claim 18, wherein the step (1) is **characterized by** sequentially adding methyl 3-mercaptopropanoate, Xantphos, DIEA (N,N-diisopropylethylamine), and Pd₂(dba)₃ to the compound represented by [Chemical Formula 7] in dioxane.

25. The method of claim 18, wherein the step (2) is **characterized by** adding 20% sodium ethoxide (EtONa) to the compound represented by [Chemical Formula 8] in dry tetrahydrofuran (THF).

26. The method of claim 18, wherein the step (3) is **characterized by** adding K₂CO₃ along with one or more selected from the group consisting of 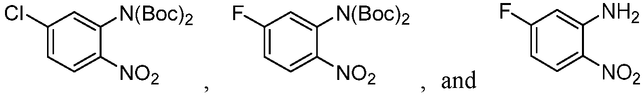 in 1-methyl-2-pyrrolidinone (NMP).

27. The method of claim 18, wherein the step (4) is **characterized by** synthesizing [Chemical Formula 11] from the compound represented by [Chemical Formula 10] and then adding 1,3-bis(methoxycarbonyl)-2-methyl 2-thiopseudoeura to prepare the compound represented by [Chemical Formula 6]. 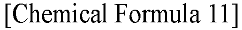 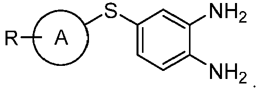 (in Chemical Formula 11,
A is a C₃-C₁₀ cyclic alkyl group, a heterocycloalkyl group, an aryl group, or a heteroaryl group, and
R is one or more selected from the group consisting of hydrogen, a halogen group, a chained or cyclic alkyl group of C₁-C₁₀, a heterocycloalkyl group, an aryl group, and a heteroaryl group,
wherein the cyclic alkyl group, the heterocycloalkyl group, the aryl group, or the heteroaryl group is unsubstituted or substituted with one or more selected from the group consisting of a C₁-C₁₀ chained alkyl group, an alkylsulfonyl group, and a carbonyl group, and
wherein the carbonyl group is substitutable with a chained or cyclic alkyl group of C₁-C₁₀, a heterocycloalkyl group, an aryl group, or a heteroaryl group.)

28. The method of claim 27, wherein the step (3) is **characterized by** adding methanol (MeOH), hydrogen gas (H₂), and palladium/carbon (Pd/C) to the compound represented by [Chemical Formula 10] to synthesize the compound represented by [Chemical Formula 11].

29. The method of claim 27, wherein the step (3) is **characterized by** adding acetic acid (AcOH) and zinc (Zn) to the compound represented by [Chemical Formula 10] to synthesize the compound represented by [Chemical Formula 11].

30. The method of claim 27, wherein the compound represented by [Chemical Formula 11] is any one selected from the group consisting of the following [Chemical Formula 11-1] to [Chemical Formula 11-6]: 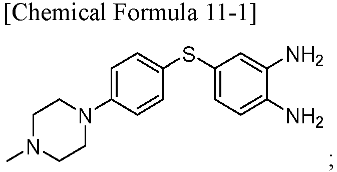 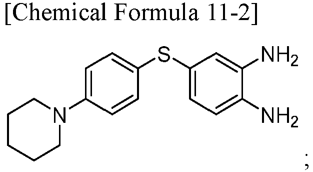 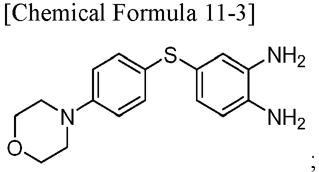 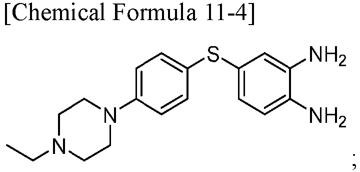 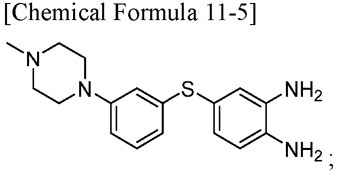 and

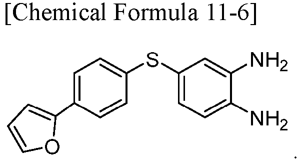

31. A compound represented by following [Chemical Formula 8]: 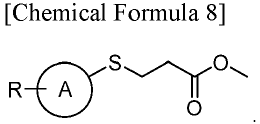 (in Chemical Formula 8,
A is a C₃-C₁₀ cyclic alkyl group, a heterocycloalkyl group, an aryl group, or a heteroaryl group, and
R is one or more selected from the group consisting of hydrogen, a halogen group, a chained or cyclic alkyl group of C₁-C₁₀, a heterocycloalkyl group, an aryl group, and a heteroaryl group,
wherein the cyclic alkyl group, the heterocycloalkyl group, the aryl group, or the heteroaryl group is unsubstituted or substituted with one or more selected from the group consisting of a C₁-C₁₀ chained alkyl group, an alkylsulfonyl group, and a carbonyl group, and
wherein the carbonyl group is substitutable with a chained or cyclic alkyl group of C₁-C₁₀, a heterocycloalkyl group, an aryl group, or a heteroaryl group.)

32. The method of claim 31, wherein
the A is one or more selected from the group consisting of a phenyl group, a furanyl group, a pyridinyl group, and a pyrimidinyl group, and the R is one or more selected from the group consisting of a piperazinyl group, a piperidinyl group, a morpholinyl group, a thiophenyl group, an imidazolyl group, a triazolyl group, and 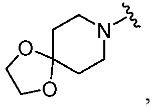 wherein the piperazinyl or piperidinyl group is unsubstituted or substituted with one or more selected from the group consisting of a methyl group, an ethyl group, a propyl group, a methylsulfone group, and

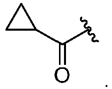

33. A method for preparing a pharmaceutically acceptable salt of a thiobenzimidazole derivative represented by [Chemical Formula 6], the method comprising the following steps:
(1) preparing a compound represented by [Chemical Formula 8] by adding methyl 3-mercaptopropanoate, Xantphos, DIEA (N,N-diisopropylethylamine), and Pd₂(dba)₃ to a compound represented by [Chemical Formula 7];
(2) preparing a compound represented by [Chemical Formula 9] by adding sodium ethoxide (EtONa) to the compound represented by [Chemical Formula 8] as prepared above;
(3) preparing a compound represented by [Chemical Formula 10] by adding one or more selected from the group consisting of 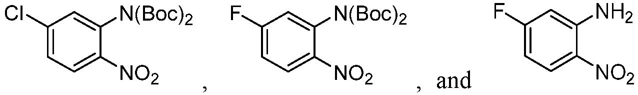 to the compound represented by [Chemical Formula 9] as prepared above;
(4) preparing a compound represented by [Chemical Formula 6] by adding 1,3-bis(methoxycarbonyl)-2-methyl 2-thiopseudoeura to the compound represented by [Chemical Formula 10] as prepared above; and
(5) preparing a hydrogen chloride (HCl) salt of the compound represented by [Formula 6] by adding HCl to the compound represented by [Formula 6] as prepared above. 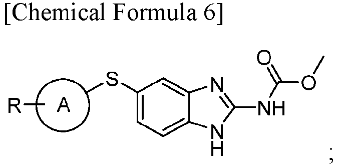 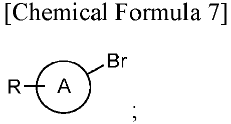 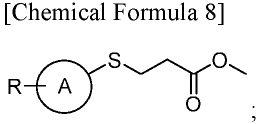 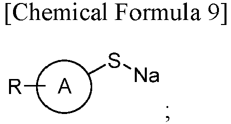 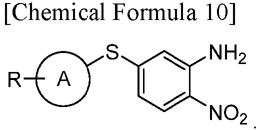 (in Chemical Formulae 6 to 10,
A is a C₃-C₁₀ cyclic alkyl group, a heterocycloalkyl group, an aryl group, or a heteroaryl group, and
R is one or more selected from the group consisting of hydrogen, a halogen group, a chained or cyclic alkyl group of C₁-C₁₀, a heterocycloalkyl group, an aryl group, and a heteroaryl group,
wherein the cyclic alkyl group, the heterocycloalkyl group, the aryl group, or the heteroaryl group is unsubstituted or substituted with one or more selected from the group consisting of a C₁-C₁₀ chained alkyl group, an alkylsulfonyl group, and a carbonyl group, and
wherein the carbonyl group is substitutable with a chained or cyclic alkyl group of C₁-C₁₀, a heterocycloalkyl group, an aryl group, or a heteroaryl group.)

34. The method of claim 33, wherein the step (5) is **characterized by** adding methanol (MeOH) and hydrogen chloride (HCl) to the compound represented by [Chemical Formula 6], and then sequentially adding isopropyl alcohol and isopropyl ether to prepare the HCl salt of the compound represented by [Chemical Formula 6].

35. The method of claim 33, wherein the step (5) is **characterized by** adding methylene chloride (CH₂Cl₂) and methanol (MeOH) to the compound represented by [Chemical Formula 6] and performing cooling, and then adding hydrogen chloride (HCl) saturated in dioxane to prepare the HCl salt of the compound represented by [Chemical Formula 6].

36. The method of claim 33, wherein the step (5) is **characterized by** adding hydrogen chloride (HCl) gas to the compound represented by [Chemical Formula 6], and then adding ethyl ether to prepare the HCl salt of the compound represented by [Chemical Formula 6].
